Europäisches Patentamt
European Patent Office
Office européen des brevets

(11) Publication number: **0 197 611 A2**

# EUROPEAN PATENT APPLICATION

(21) Application number: **86200579.0**

(51) Int. Cl.⁴: **C07C 85/06**

(22) Date of filing: **04.04.86**

(30) Priority: **04.04.85 US 720154**

(43) Date of publication of application: **15.10.86 Bulletin 86/42**

(84) Designated Contracting States: **BE DE FR GB IT NL SE**

(71) Applicant: **UNION CARBIDE CORPORATION**
**39 Old Ridgebury Road**
**Danbury Connecticut 06817(US)**

(72) Inventor: **Gibson, Charles Arnold**
**2910 Macon Street So.**
**Charleston West Virginia 25303(US)**
Inventor: **Doumaux, Arthur Roy, Jr.**
**1401 Wilkie Drive**
**Charleston West Virginia 25314(US)**
Inventor: **Schreck, David James**
**5226 Sun Valley Drive**
**Cross Lanes West Virginia 25313(US)**

(74) Representative: **Smulders, Theodorus A.H.J. et al**
**Vereenigde Octrooibureaux Nieuwe Parklaan 107**
**NL-2587 BP 's-Gravenhage(NL)**

## (54) Conversion of oxygen-containing polyamines.

(57) Process for preparing polyalkylene polyamines by contacting an alkanolamine, such as, ethanolamine, and a reactive nitrogen-containing base which is ammonia or a primary or secondary amine, in a first reactor to produce an alkyleneamine, such as, ethylenediamine, and an aminoalkylalkanolamine, such as, aminoethylethanolamine, in the presence of a catalytically effective amount of an amination catalyst and at a temperature and pressure sufficient to form the alkyleneamine and aminoalkylalkanolamine. Then the alkyleneamine, aminoalkylalkanolamine, unreacted alkanolamine and unreacted nitrogen-containing base are reacted in a second reactor to produce the polyalkylene polyamines, such as, diethylenetriamine, in the presence of a catalytically effective amount of an amination catalyst, such as, a reductive amination catalyst or a phosphorus-containing catalyst, and at a temperature and pressure sufficient to form the polyalkylene polyamines. Preferably the phosphorus-containing catalyst is a Group IIIB metal acid phosphate. By controlling the relative amounts of the feed components, one can achieve a desired molecular weight distribution and range in the polyalkylene polyamine product.

FIG. 1

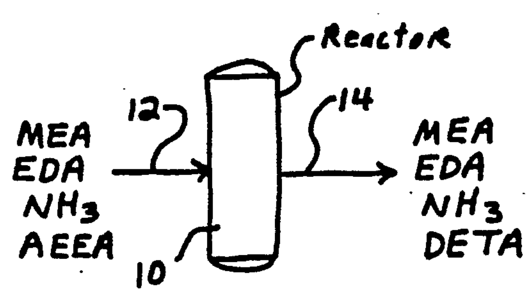


EP 0 197 611 A2

# CONVERSION OF OXYGEN-CONTAINING POLYAMINES

## BACKGROUND OF THE INVENTION

The invention relates to the preparation of polyalkylene polyamines.

U. S. Patent No. 4,463,193 discloses a process for preparing predominantly noncyclic polyalkylene polyamines. Ammonia or a primary or secondary amine is contacted with an alkanolamine compound having an amino group and a primary or secondary hydroxy group and an alkyleneamine compound having two amino groups in the presence of a catalytically effective amount of a Group IIIB metal acid phosphate. A temperature is used which is sufficient to effect a reaction among the ammonia or amine, the alkanolamine compound and the alkyleneamine compound under a pressure sufficient to maintain a substantial amount of the ammonia or amine in the reaction zone. The highest conversion shown in the examples of U.S. Patent No. 4,463,193 is 43 percent.

U. S. Patent Nos. 4,111,840 and 4,123,462 disclose a process for producing ethylenediamine by the catalytic amination of monoethanolamine. The ethanolamine is reacted with ammonia in the presence of hydrogen and a Ni-Re catalyst comprising rhenium and nickel impregnated on a support material, which is alumina, silica, silica-alumina, diatemaceous earth or silica-titania. The temperature of the amination reaction is in the range of 125° to 350°C., the pressure is 500 to 5,000 p.s.i.g. and the amount of ammonia present is in excess of two times the stoichiometric amount required by the ethanolamine.

U. S. Patent No. 4,209,424 discloses a catalytic process in a heterogeneous phase for producing ethylenediamine and piperazine from ethanolamine and ammonia. The ethanolamine-ammonia reaction in the presence of the amination catalyst is conducted at a temperature between 170° and 260°C. and at a pressure between 50 and 300 bars absolute. The ethanolamine, ammonia, and hydrogen are introduced into the reactor in quantities such that the ammonia-ethanolamine molar ratio is between 5 and 40 and the hydrogen flow rate is between 5 and 200 nl per mole of ethanolamine. The amination catalyst has at least one active metal from the group of transition metals consisting of nickel, cobalt and copper, uniformly combined with a refractory microporous substance.

U.S. Patent No. 4,314,083 discloses a process for selectively preparing predominantly noncyclic polyalkylene polyamine compounds which are disclosed wherein an alkylene polyamine compound is contacted with an hydroxy compound in the presence of a catalytically effective amount of a salt of a nitrogen or sulfur containing substance or the corresponding acid at a temperature of from 250° to 300°C. under a pressure sufficient to maintain the reaction mixture essentially in liquid phase. The polyalkylene polyamine thus formed is recovered from the reaction mixture.

U.S. Patent No. 4,362,886 teaches a process for selectively preparing predominantly noncyclic polyalkylene polyamine compounds which is disclosed wherein an alkylene polyamine compound is contacted with a hydroxy compound in the presence of a catalytically effective amount of a substance of arsenic, antimony or bismuth at a temperature of from 250° to 300°C. under a pressure sufficient to maintain the reaction mixture essentially in liquid phase. The polyalkylene polyamine thus formed is recovered from the reaction mixture.

## BROAD DESCRIPTION OF THE INVENTION

The invention provides processes for preparing polyalkylene polyamines wherein the selectivity of noncyclics to cyclics is excellent and can be controlled and varied. The invention also provides processes for preparing polyalkylene polyamines wherein the molecular weight distribution and range can be controlled.

Broadly the invention involves a process for preparing polyalkylenepolyamines from a feed composed of (i) an alkanolamine having at least one amino group, (ii) an alkyleneamine having at least two amino groups, (iii) an oxygenated organic amine compound other than the alkanolamine (i), and (iv) a reactive nitrogen-containing base selected from the group consisting of ammonia or a primary amine or a secondary amine. The alkanolamine, alkyleneamine, oxygenated amine compound and said reactive nitrogen-containing base are contacted in a reaction zone to produce the polyalkylene polyamines in the presence of a an amination catlyst, such as, catalytically effective amount of a reductive amination catalyst or a phosphorus-containing catalyst, and at a temperature and pressure sufficient to form the polyalkylene polyamines.

The invention more preferably involves a process for preparing polyalkylene polyamines from a feed composed of (i) an alkanolamine, (ii) an alkylenediamine, (iii) aminoalkylalkanolamine and - (iv) a reactive nitrogen-containing base selected from the group consisting of ammonia, a primary amine and a secondary amine. The alkanolamine, alkylenediamine, aminoalkylalkanolamine and reactive nitrogen-containing base are contacted in a reaction zone to produce the polyalkylene polyamines in the presence of a catalytically effective amount of a reductive amination catalyst or a phosphorus-containing catalyst and at a temperature and pressure sufficient to form the polyalkylene polyamines. Typically the alkanolamine is ethanolamine, the alkylenediamine is ethylenediamine, the aminoalkylalkanolamine is aminoethylethanolamine and ammonia is used. The process converts the aminoethylethanolamine with ammonia into diethylenetriamine and with ethylenediamine into triethylenetetramine -both products are valuable commodities. The AEEA is also converted to piperazine.

The reaction is preferably conducted in the vapor phase or the supercritical phase, but can be conducted in the liquid phase. Preferably the liquid hourly space velocity of the reactants in the reaction is between 1 and 25 per hour. The reaction most preferably is conducted at a temperature between about 260° and 300°C. when a phosphorus-containing catalyst is used, and is preferably conducted at a temperature of 150° to 235°C. when a reductive amination catalyst is used. The reaction pressure is preferably between about 200 and 2,000 p.s.i.g. A gaseous diluent can be used in the reactor.

The invention also broadly includes a process for preparing polyalkylene polyamines which includes contacting (i) an alkanolamine having at least one amino group and (ii) a reactive nitrogen-containing base selected from the group consisting of ammonia, a primary amine and a secondary amine, and/or an alkyleneamine having at least two amino groups, in a first reaction zone to produce an alkyleneamine having at least two amino groups, and an oxygenated organic amine compound other than said alkanolamine (i), in the presence of a catalytically effective amount of a reductive amination catalyst and at a temperature and pressure sufficient to form the alkyleneamine and the oxygenated organic amine compound and at least a part of said reaction product stream (b) comprising alkyleneamine, oxygenated organic amine compound, unreacted alkanolamine and unreacted reactive nitrogen-containing base are contacted in another reaction zone to produce the polyalkylene polyamines in the presence of a catalytically effective amount of an amination catalyst, such as, a reductive amination catalyst or a phosphorus-containing catalyst, and at a temperature and pressure sufficient to form the polyalkylene polyamines.

The invention preferably involves a process for preparing polyalkylene polyamines which includes first contacting (i) an alkanolamine having at least one amino group and (ii) a reactive nitrogen-containing base selected from the group consisting of ammonia, a primary amine and a secondary amine and/or an alkyleneamine having at least two amino groups, in a first reaction zone to produce an alkyleneamine having at least two amino groups and an aminoalkylalkanolamine in the presence of a catalytically effective amount of a reductive amination catalyst and at a temperature and pressure sufficient to form the alkyleneamine and said aminoalkylalkanolamine. Typically the alkanolamine is ethanolamine, the alkyleneamine is ethylenediamine, ammonia is used and the aminoalkylalkanolamine is aminoethylethanolamine. The aminoalkylalkanolamine, alkyleneamine, unreacted alkanolamine and unreacted reactive nitrogen-containing base are contacted in another reactor to produce the polyalkylene polyamines in the presence of a catalytically effective amount of an amination catalyst, such as, reductive amination catalyst or a phosphorus-containing catalyst, and at a temperature and pressure sufficient to form the polyalkylene polyamines. All or part of the feed from the first reactor can be fed to the second reactor.

The use of two reactors in series allows the economical production of polyalkylene polyamines having at least four carbon atoms without the normal problems encountered by the prior art processes. Whereas U.S. Patent No. 4,463,193 specifically discloses achieving a maximum of 43 percent conversion in its examples, this invention's use of two reactors in tandem can often provide conversions of 80 mole percent or higher. The reaction feed from the first reactor to the second or another reactor contains alkyleneamine(s), such as, ethylenediamine, plus alkanolamines, such as, aminoethylethanolamine. The use of the two reactors in series saves on energy and equipment because the alkyleneamine does not have to be refined to remove MEA and ammonia before it is fed into the second reactor. Of course, not all of the reaction stream from the first reactor has to be fed into the second reactor -the diverted portion of such reaction stream could be refined to remove, for example, the alkyleneamine. If desired, for example, a separator or reactor can be used between the two noted reactors.

A higher quality polyalkylene polyamine product is obtained if some of the alkyleneamine is consumed in the second reactor as opposed to running the second reactor in a mode of zero

consumption of the alkyleneamine. Hydrogen can be used in the second reactor or both reactors to provide high quality polyalkylene polyamine products.

Preferably the first reaction is conducted in the vapor phase or the supercritical phase, but can be conducted in the liquid phase. A gaseous diluent can be added to the first reactor.

In the first reactor, preferably the mole ratio of the ammonia to the alkanolamine is at least 20:1.25 to 0.6:20, typically between 20:1 and 0.6:20, as this provides higher selectivity of the noncyclic polyalkylene polyamines over the cyclic polyalkylene polyamines in the final product. The first reaction is conducted preferably at a temperature of 150° to 235°C. and most preferably at a pressure between 200 and 2,000 p.s.i.g. Preferably the liquid hourly space velocity of the reactants in the first reactor is between 1 and 25 per hour.

The catalyst used in the first reactor is a reductive amination catalyst, most preferably a nickel-rhenium -boron catalyst comprising rhenium, boron and nickel impregnated on a support material which is alumina, silica, silica-alumina, diatomaceous earth or silica-titania.

The conditions and catalysts used in the second catalyst in the series is the same as those for the just-above described first reactor of this embodiment of the invention. Preferably the second reactor is run at a lower pressure than in the first reactor. When a phosphorus-containing catalyst is used in the second reactor, the temperature is most preferably between 260° and 300°C.

The invention also involves a process for affecting the molecular range and distribution of polyalkyline polyamines prepared from a feed composed of an alkanolamine and ammonia or a primary amine or a secondary amine. The process uses the above-described system of using two reactors in series. The product feed from the second reactor is sent to a separation stage where the alkyleneamine is separated as a separate component, the alkanolamine is separated as a separate component and the polyalkylene polyamines are separated as a separate component.

By recycling at least part of the separated alkanolamine to the first reactor the molecular range and distribution of the polyalkylene polyamines produced in the second reaction can be affected as desired. A high ratio of monoethanolamine to ammonia in the first reactor increases the production of ethylenediamine in the first reactor. Likewise, by recycling at least part of the separated alkyleneamine to the first reactor, the molecular range and distribution of the polyalkylene polyamines produced in the second reaction can be affected as desired. A high ethylenediamine feed to the second reactor increases the production of diethylenetriamine in the second reactor. Feeding diethylenetriamine to the second reactor provides a broader range of polyalkylene polyamines produced.

Any suitable separation method can be used, but preferably the separation is conducted using distillation. Most preferably the separation is conducted using a fractional distillation column with the polyethylene polyamines coming off of the bottom of the column and with the alkanolamine and the alkyleneamine coming off in the top portion of the column as separate components.

The recycle of the alkanolamine and/or alkyleneamine allows the tailor-making of the polyalkylene polyamine product as concerns its molecular weight distribution and range. Such recycling also allows the consumption of at least some of the EDA in the second reactor of the EDA which has been produced in the first reactor -this EDA consumption mode produces a better quality product over a no-EDA consumption mode. The use of the two-reactors-in-series scheme allows a total conversion of 80 to 90 mole percent of the starting alkanolamine, and provides high selectivity between DETA and PIP. The recycle of EDA to the second reactor provides increased conversion of MEA in the second reactor.

The invention also broadly involves a process for affecting the molecular range and distribution of polyalkylene polyamines, which involves contacting (i) an alkyleneamine having at least two amino groups, (ii) an alkanolamine having at least one amino group, and/or a reactive nitrogen-containing base selected from the group consisting of ammonia, a primary amine and a secondary amine, and (iii) a stream containing an oxygenated organic amine compound other than the alkanolamine, in a reaction zone in the presence of a catalytically effective amount of an amination catalyst, such as, a reductive amination catalyst, or a phosphorus containing catalyst and at a temperature and pressure sufficient to form the polyalkylene polyamines. If desired, at least a part of the reaction stream can be recycled to serve as feed to the reactor. The alkyleneamine as a separate component, the alkanolamine as a separate component, a separate feed containing the oxygenated organic amine compound, and/or the polyalkylene polyamines as a separate component can be separated from the reaction stream. At least part of the separated alkanolamine can be recycled to the reaction zone to affect the molecular range and distribution of said polyalkylene polyamines product in the reaction. Also, at least part of the separated alkyleneamine can be recycled to the reaction zone to affect the molecular range and distribution of the polyalkylene polyamines produced in the reaction as desired. Further, at least part of the separated stream of containing said oxygenated organic amine compound can be recycled to the reaction zone to affect the molecular range and distribution of the polyalkylene polyamines produced in the reaction.

The oxygenated organic amine is preferably an aminoalkylalkanolamine, which preferably is aminoethylethanolamine. When AEEA is recycled, often 2 to 20, preferably 5 to 10, mole percent, based on the MEA feed, of the AEEA is recycled to produce di-and tri-poly-ethylene polyamines.

The feed for the above reactor can be obtained from another reactor wherein (i) an alkanolamine having at least one amino group and (ii) a reactive nitrogen-containing base selected from the group consisting of ammonia or a primary amine or a secondary amine, and/or an alkyleneamine having at least two amino groups, are contacted, to produce an alkyleneamine having at least two amino groups and an oxygenated organic amine compound (which is different than the alkanolamine), such as, an aminoalkylalkanolamine, in the presence of a catalytically effective amount of a reductive amination catalyst and at a temperature and pressure sufficient to form the alkyleneamine and the oxygenated organic amine compound.

A feed containing an oxygenated organic amine compound can be fed to the first or second or both of the reactors. Such a feed can be separated from or can be the reaction product stream from the second reactor.

The invention further involves a process for obtaining a specified molecular range and distribution of polyalkylene polyamines prepared from a feed composed of (i) an alkanolamine having at least one amino group, (ii) an alkyleneamine having at least two amino groups, (iii) a reactive nitrogen-containing base selected from the group consistng of ammonia, a primary amine and a secondary amine and (iv) an oxygenated organic amine compound, such as, aminoalkylalkanolamine. The alkanolamine, alkyleneamine, oxygenated organic amine compound and reactive nitrogen-containing base are contacted to produce the polyalkylene polyamines in the presence of a catalytically effective amount of an amination catalyst, such as, a reductive amination catalyst or a phosphorus-containing catalyst, and at a temperature and pressure sufficient to form the polyalkylene polyamines. Before and/or during the reaction step, the amount of the alkanolamine and alkyleneamine in the feed, relative to each other, are adjusted to provide the specified molecular range and distribution of the polyalkylene polyamines formed in the reaction. The oxygenated organic amine compound can be likewise added.

In this four component feed embodiment, when the MEA content is lowered and the EDA content is raised, more DETA is produced. When the MEA is increased and the EDA is lowered, more AEEA is produced. When the $NH_3$ is increased and the other components are held steady, more EDA and DETA are produced. When the AEEA content is increased and the other components are held steady, the amount of TETA produced is increased. As more DETA is produced, more AEEA and PIP are produced. As the space velocity is increased, the conversion to PIP is slowed down. As the reaction temperature is increased, more PIP is produced.

The invention also includes a process for the production of higher molecular weight polyalkylene polyamines from a feed which includes oxygen-containing higher molecular weight polyalkylene polyamines. Such feed containing the oxygen-containing higher molecular weight polyalkylene polyamines is contacted with an alkyleneamine having at least two amino groups in the presence of a catalytically effective amount of an amination catalyst, such as, a reductive amination catalyst or a phosphorus-containing catalyst, at a temperature and pressure effective to produce the higher molecular weight weight polyalkylene polyamines. Preferably higher molecular weight polyethylene polyamines having at least four amino groups which are produced.

## BRIEF DESCRIPTION OF THE DRAWINGS

In the drawings:

Figure 1 is a schematic diagram of a preferred embodiment of the process of the invention;

Figure 2 is a schematic diagram of another preferred embodiment of the process of the invention; and

Figure 3 is a schematic diagram of a further preferred embodiment of the invention.

## DETAILED DESCRIPTION OF THE INVENTION

As used herein, all parts, percentages, ratios and proportions are on a weight basis and all temperatures are in degrees Centigrade, unless otherwise stated or otherwise obvious herefrom.

Also as used herein, the term "ethanolamine" specifically means monoethanolamine unless otherwise indicated or implied herein. U.S. Sieve Series mesh sizes are used herein.

The alkyleneamine having at least two amino groups is preferably an unbranched alkylene moiety, such as, ethylenediamine, and preferably has primary amino groups. The alkanolamine preferably has a primary or secondary hydroxyl moiety and preferably amino group(s). Preferably, the alkanolamine has an unbranched alkylene moiety.

The alkanolamine compounds used in the invention process include those represented by the formula:

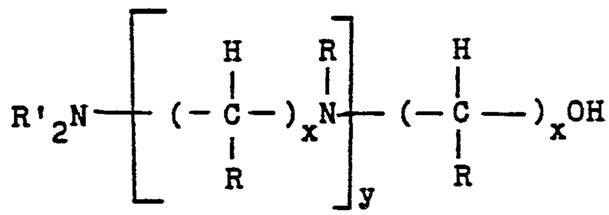

wherein R is hydrogen or a lower alkyl ($C_1$ to $C_4$) radial, R' is hydrogen or an alkyl ($C_1$ to $C_{25}$) radical, x is a number from 2 to 6, and y is a number from 0 to 3. Examples of suitable alkyl radicals are the lower ($C_1$ to $C_4$) alkyls, such as, methyl, ethyl and butyl, and higher alkyls, such as, octyl, decyl and octadecyl. Methyl is the preferred lower alkyl radical. However, it is preferred that R and R' both are hydrogen; thus the alkanolamine would contain a primary amino group. Examples of useful alkanolamine compounds are the ethanolamines, isomeric propanolamines, N-(2-aminoethyl) ethanolamine, N-methylethanolamine, N,N-dimethylethanolamine, N,N,N'-trimethylaminoethylethanolamine and the like. Recycle of at least a portion of the unreacted alkanolamine recovered from the product stream to the first reactor allows control of the molecular weight distribution and range of the produced polyalkylene polyamines.

The alkyleneamine reactants used in the invention process are represented by the formula:

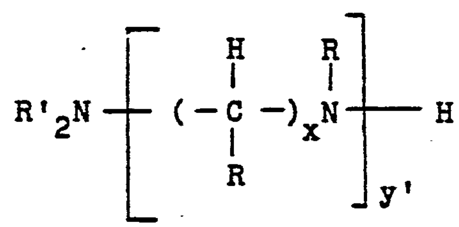

wherein R is hydrogen or a lower alkyl ($C_1$ to $C_4$) radical, R' is hydrogen or an alkyl ($C_1$ to $C_{25}$) radical, x' is a number from 2 to 6, and y' is a number from 1 to 4. Examples of suitable alkyl radicals are the lower ($C_1$ to $C_4$) alkyls, such as, methyl, ethyl and butyl, and higher alkyls, such as, octyl, decyl and octadecyl. It is preferred that R and R' are both hydrogen. The preferred lower alkyl radical is methyl. Examples of useful alkyleneamine compounds are 1,3-propylenediamine, N-methylpropylenediamine, 1,2-propylenediamine, diethylenetriamine, tributylenetetraamine, triethylethylenetetraamine, N,N,N'-trimethyldiethylenetriamine, noncyclic isomers of triethylenetetramine, noncyclic isomers of tetraethylenepentamine, N-methylethylenediamine, N,N-dimethylethylenediamine and ethylenediamine, which is the preferred alkyleneamine compound. Recycle of at least a portion of the alkylenediamine separated from the product stream to the second reactor allows control of the molecular weight distribution and range of the produced polyalkylene polyamines.

Oxygenated organic amine compounds include organic amine compounds containing at least one hydroxyl group and/or at least one ether group. The described alkanolamines are included with the oxygenated organic amine compounds as long as the oxygenated organic amine compounds are different therefrom when one of such alkanolamines

are expressly specified as a feed member. The preferred oxygenated organic amine compounds are the aminoalkylalkanolamines, such as, aminoethylethanolamine.

The oxygenated organic amine compounds can be compounds having the formula:

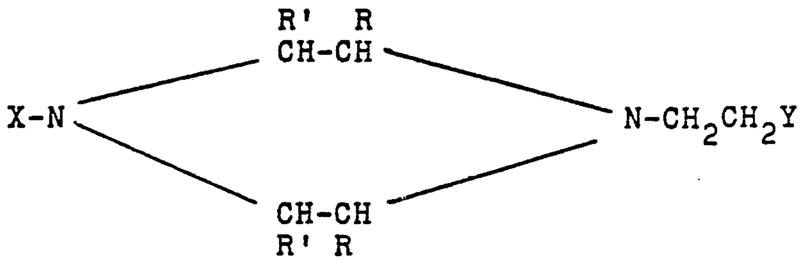

or compounds having the formula:

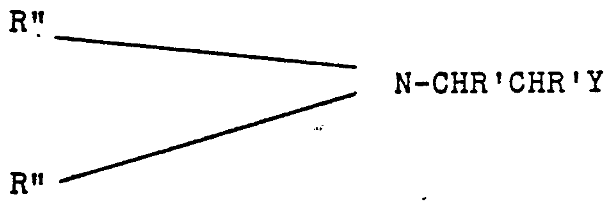

wherein R is hydrogen, an alkyl group containing 1 to 12 carbon atoms or a cycloalkyl group containing 6 to 12 carbon atoms, R' is hydrogen or an alkyl group containing 1 to 4 carbon atoms, R" is hydrogen or -CHR'CHR'Y, X is hydrogen or -$CH_2CH_2Y$ and Y is -OH.

Examples of the oxygenated organic amine compounds are aminoethylethanolamine, N-(2-hydroxyethyl)piperazine, N,N-di(hydroxyethyl)-piperazine, N-(hydroxy ethyl)-2-methylpiperazine, N,N',-di(hydroxyethyl-2-methylpiperazine, N-(hydroxyethyl)-2-ethylpiperazine, N,N'-di-(hydroxyethyl)-2-ethylpiperazine, N-(hydroxyethyl)-2-butylpiperazine, N,N-di(hydroxyethyl)-2-butylpiperazine, N-(hydroxyethyl)-2-dodecylpiperazine, N-(hydroxyethyl)-2-cyclohexylpiperazine, N-(hydroxyethyl)-2-hexylcyclohexylpiperazine, N,N'-di(hydroxyethyl)-2,5-dimethylpiperazine, N-(hydroxyethyl)-2,3,5,6-tetramethylpiperazine, N,N'-di(hydroxyethyl)-2,5-dimethylpiperazine, N-(hydroxyethyl)-2,5-diethylpiperazine, aminopropylethanolamine, aminopropylpropanolamine, N-(hydroxyethyl)diethylenetriamine, N-(hydroxypropyl)diethylenetriamine, N-2-hydroxybutyl)diethylenetriamine, N-(hydroxyethyl)-dipropylenetriamine, N-(hydroxypropyl)-dipropylenetriamine, N-(2-hydroxybutyl)-dipropylenetriamine and morpholine.

Ammonia and the preferred primary and secondary amines which are used in the invention process are represented by the formula:

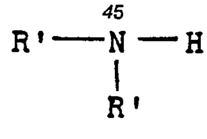

wherein R' is hydrogen or an alkyl ($C_1$ to $C_{25}$) radical, preferably a lower alkyl ($C_1$ to $C_4$) radical, such as methyl or ethyl. Useful amine feestocks include monomethylamine, dimethylamine, monoethylamine, diethylamine, octylamine and octadecylamine. The ammonia or amine can act as both a reactant and a diluent (when excess of it is present) in the invention process. When ammonia is used, the mole ratio of ammonia (or the primary amine or the secondary amine) to the reactants is usually in the range of 20:1 to 0.6:20 and preferably in the range of 20:1.25 to 1:1.

Noncyclic polyalkylene polyamines that are produced by the reaction of (a) alkanolamine and alkylenediamine or, (b) ammonia, an alkyleneamine and an alkanolamine or (c) ammonia and an alkanolamine are represented by the formula:

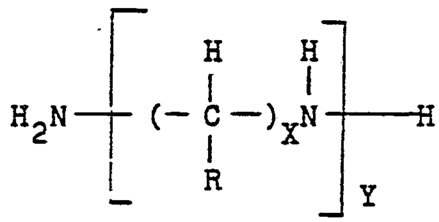

wherein R is hydrogen or a lower alkyl ($C_1$ to $C_4$) radical, preferably a methyl radical, X is a number from 2 to 6, Y is a number from 2 to 7, and X can vary for a given value of Y. Examples of such noncyclic polyalkylene polyamines that are produced are dipropylenetriamine, tributylenetetramine, di(2-methylethylene)triamine, tri(2-methylethylene)tetramine, N-(2-aminoethyl)1,3-propylenediamine, diethylenetriamine, the noncyclic isomers of triethylenetetramine and the noncyclic isomers of tetraethylenepentamine.

Noncyclic polyalkylene polyamines include polyalkylene polyamines having straight-chained and branched alkylene groups.

Cyclic polyalkylene polyamines that are produced by the reaction of (a) an alkyleneamine and an alkanolamine or (b) ammonia, an alkyleneamine and an alkanolamine or (c) an alkanolamine and ammonia are represented, for example by the following formula:

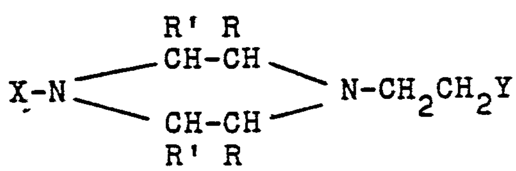

wherein R is hydrogen, an alkyl group containing 1 to 12 carbon atoms or a cycloalkyl group containing 6 to 12 carbon atoms, R' is hydrogen or an alkyl group containing 1 to 4 carbon atoms, X is hydrogen or $-CH_2CH_2Y$ and Y is -OH or $-NH_2$.

Examples of cyclic polyalkylene polyamines are pierazine, N-(2-hydroxyethyl)piperazine, N,N-di-(hydroxyethyl)piperazine, N-(hydroxyethyl)-2-methylpiperazine, N,N,'-di(hydroxyethyl-2-methyl piperazine, N-(hydroxyethyl)-2-ethylpiperazine, N,N'-di(hydroxyethyl)-2-ethylpiperazine, N-(hydroxyethyl)-2-butylpiperazine, N,N-di-(hydroxyethyl)-2-butylpiperazine, N-(hydroxyethyl)-2-dodecylpiperazine, N-(hydroxyethyl)-2-cyclohexylpiperazine, N-(hydroxyethyl)-2-hexycyclohexylpiperazine, N,N'-(dihydroxyethyl)-2,5-dimethylpiperazine, N-(hydroxyethyl)-2,3,5,6-tetramethylpiperazine, N,N'-di(hydroxyethyl)-2,5-dimethylpiperazine, N-(hydroxyethyl)-2,5-diethylpiperazine, N-(hydroxyethyl)diethylenetriamine, N-(hydroxypropyl)diethylenetriamine, N-(2-hydroxybutyl)dipropylenetriamine and morpholine.

The phrase "predominantly noncyclic polyalkylene polyamines" is meant to mean that such polyalkylene polyamines are mostly of the noncyclic species.

Use of secondary amines instead of ammonia leads to polyalkylene polyamines containing terminal dialkylamino groups. Alternatively, use of primary amines instead of ammonia leads to polyamines which contain randomly distributed monoalkylamino groups.

Generally, the mole ratio of the alkyleneamine to the alkanolamine can range from about 0.05:1 to 12:1, preferably is about 4:1 to 1:4, and most preferably is about 1:1.

In the reactor, the temperature for the reaction depends upon the particular starting material, ratios of

reactants, and most importantly, the activity of the catalyst used. In the process of the invention, generally the reaction temperature is within the range of 125° to 425°C. When a reductive amination catalyst is used, preferably the reaction temperature range is 150° to 235°C. When a phosphorus-containing catalyst is used, the reaction temperature is preferably 220° to 350°C. and most preferably 260° to 300°C.

The pressure at the time of reaction should normally be within the range from about 50 to about 4,000 p.s.i.g., preferably greater than 100 p.s.i.g. and most preferably from about 200 to about 2,000 p.s.i.g. The reaction is best conducted at a temperature high enough to keep the reactants above their dew point. This may also mean at a pressure (first defined by the temperature) which expresses the first quadrant using the critical point of the origin. Normally a diluent, such as, hydrogen, methane, water, nitrogen, helium and argon, can be added to increase the pressure in a batch reactor and the volumetric flow in a fixed bed reactor.

The reaction is preferably conducted in the vapor phase or the supercritical phase, although the reaction can be conducted in the liquid phase. The liquid hourly space velocity of the reactants is between about 0.1 and about 100 per hour and preferably between about 1 and about 25 per hour.

By reaction zone is meant that vessel, e.g., autoclave, continuous stirred tank reactor or packed bed reactor, in which the catalyst is located and production of polyalkylene polyamines is effected.

Although the reactions can be carried out in the batch mode, they are preferably conducted as continuous processes through a bed of particulate catalyst, for example, operation of a continuously stirred tank reactor or a packed (fixed) bed reactor. The continuous process is carried out by employing conventional process techniques and apparatus well known to those skilled in the art. In the continuous reaction processes, the catalyst can be added alone or in combination with the reactants, or, as stated above, the catalyst can be provided as a fixed bed on conventional support materials well known to those skilled in the art. The reaction is allowed to proceed until a desired conversion is obtained or the reaction is complete. Normally the reaction is carried out within about 0.5 to 5 hours in the batch mode or residence times (based on the ethylenediamine and ethanolamine components) of 0.1 to 4.0 hours in a continuous mode for practical levels of polyalkylene polyamine production.

The reactor can be an up-flow or down-flow reactor and can have a fluidized bed or, most commonly, a fixed bed. The catalyst bed can contain inert particles which can be interspersed throughout the bed and/or form discrete layers, e.g., at an end or intermediary to the bed. The volume of the reaction zone containing such inert particles is the reaction zone volume for purposes of determining the feed rate. Preferably, the space velocity should not be so high that for the reactor geometry, a significant amount of backmixing occurs. Advantageously, the flow through the catalyst bed is substantially plug-type flow.

The catalysts used in the invention are heterogeneous catalysts. The catalysts are used in an amount of 0.1 to 12 weight percent, preferably 0.5 to 10 weight percent, and most preferably 2 to 7 weight percent, based on the total weight of the reactants.

Any suitable or conventional reductive amination catalyst can be used in the first and/or second reactor. When a reductive amination catalyst is used, the reactor feed can include hydrogen. Preferably 4 to 12 mole percent, typically about 2 to 20 mole percent, based on the total moles of one of the reactants, preferably the most prevalent nitrogen-containing reactant, e.g., ammonia, is used.

Reductive amination catalysts are well known in the art and usually comprise the metal or oxide or one or more of nickel, copper, cobalt, iron and the like as the active species. Other compounds which can find use in such catalysts include the metal, oxide or salt of one or more of chromium, lanthanum, lithium, potassium, cesium, cerium, ruthenium, rhodium, palladium, platinum, rhenium, iridium, silver, zinc, titanium, manganese and boron. Often the catalysts are of the Raney nickel-type or Raney cobalt-type, or are supported catalysts. Many of the catalysts are preferably activated at elevated temperature in a hydrogen atmosphere. Particularly desirable catalysts comprise nickel as the catalytically-active species.

The preferred reductive amination catalysts are those which are nickel on a catalyst support material and the most preferred are those which are a mixture of nickel and rhenium impregnated on various support materials including alumina, silica, silica-alumina, kieselguhr or diatomaceous earth and silica-titania. Preferably the mole ratio of the nickel to the rhenium is the range of from 2:1 to about 30:1 and the total nickel and rhenium metal

present is in the range of 3 to 30 percent by weight of the support. Such catalysts can be prepared by the methods taught in U.S. Patent Nos. 4,111,840 and 4,123,462, the pertinent parts of which are incorporated herein by reference. Basically, such catalysts are solid catalysts wherein the nickel and rhenium metals are supported on certain catalyst support materials.

Hydrogen is used in the reactor to maintain the activity of the nickel-rhenium catalyst (and has a diluent effect in the reactor zone). Typically, hydrogen is provided in an amount of at least 2 mole percent based on the total moles of ammonia and often this percentage is between about 2 to 20 and preferably is about 4 to 12 mole percent.

The nickel-rhenium catalyst can contain various other metals in admixture with the nickel and rhenium which do not detrimentally affect the catalytic properties of catalysts containing nickel and rhenium as the only impregnated metals. These additional metals, in certain amination processes, can actually improve selectivity and activity of the basic Ni-Re catalyst. Certain of these metals can extend the activity life and other physical properties of the Ni-Re catalyst. Examples of catalysts containing additional metal components include Ni-Re-La, Ni-Re-Ca, Ni-Re-Mg, Ni-Re-Sr, Ni-Re-Li, Ni-Re-K, Ni-Re-Ba, Ni-Re-Ce, Ni-Re-W, Ni-Re,Fe, Ni-Re-Ru, Ni-Re-Cu, Ni-Re-Ag, Ni-Re-Zn, Ni-Re-Co, Ni-Re-U, Ni-Re-Ti and Ni-Re-Mn.

The amount of Ni-Re catalyst present in the process depends on many variables including the reactants, the relative proportions of the reactants, reaction condition and the degree of conversion and selectivity desired. Moreover, the amount of catalyst will depend also on the nature of the catalyst itself, e.g., its metal loading and activity and age. The catalyst must be present in the reaction zone in sufficient catalytic amount to enable the desired reaction to occur. This is also true for any of the catalysts useful in the invention process.

Other preferred reduction amination catalysts are catalyst composed of rhenium, nickel and boron impregnated on a support material selected from the group consisting of aluminas (e.g., alpha), silicas, silica-aluminas, kieselguhrs or diatomaceous earths and silica-titanias, wherein the ratio of nickel to boron to rhenium is in the range of from about 2:2:1 to about 30:30:1 and the total nickel, boron and rhenium present is in the range of about 3 to about 30 percent by weight of the support material.

The most preferred mode of the catalyst is a reductive amination catalyst which has a silica support marketed under the mark T-869® by United Catalyst Incorporated. The best mode of the catalyst is obtained from the United Catalyst Incorporated. It is believed that the catalyst is prepared by impregnated the T-869® catalyst support with an aqueous solution containing nickel nitrate, ammonium perrhenate and boric acid in a metal composition of 66 percent of nickel, 19.6 percent of rhenium and 14.4 percent of boron. Following the impregnation, it is calcined at 330°C. for 2.5 hours. Then it is impregnated a second time with the same solution and calcined again under the same conditions for the same length of time. Thereafter it is impregnated a third time to reach a 12 percent total metal loading. It is then calcined at 330°C. for 2.5 hours. Then it is reduced under a hydrogen atmosphere at a temperature of 332° to 337°C. for 16 hours. Following the reduction step it is stabilized at 60°C. for 30 to 40 hours.

Examples of other useful reductive amination catalysts are the rhodium atom-containing catalysts of U.S. Patent No. 4,322,530, the copper-rhenium catalysts of U.S. Patent No. 4,206,149, the nickel-cobalt-iron catalysts of U.S. Patent No. 3,766,184, the cobalt-nickel-copper-containing aluminum oxide or silicon dioxide support catalyst of U.S. Patent No. 4,014,933 and the catalysts containing copper oxide or copper hydroxide, nickel oxide or nickel hydroxide, and, optionally, an oxide or a hydroxide of a Group IIA metal of U.S. Patent No. 4,409,399 - the pertinent parts of such patents are incorporated herein by reference. U.S. Patent No. 4,209,424 (the pertinent parts of which are incorporated herein by reference) teaches an amination catalyst which has at least one active metal from the group of transition metals consisting of nickel, cobalt and copper, uniformly combined with a refractory microporous substance.

The pertinent parts of copending, commonly-assigned U.S. Patent Application Serial No. 454,485, filed on December 29, 1982, are incorporated herein by reference. U.S. Ser. No. 454, 485 discloses a process for the production of an amine composition having a high ratio of diethylenetriamine to piperazine which comprises maintaining ethylenediamine in the presence of a nickel, cobalt or rhodium catalyst, wherein the metal is present on the surface of the catalyst in a polyatomic form, and at a temperature between about 170° to about 210°C. sufficient to convert less than about 35 percent of the ethylenediamine feed. The catalysts of U.S. Ser. No. 454,485 are nickel, cobalt or rhodium catalysts which can be relatively pure metal catalysts or catalysts that

have been modified by the addition of molybdenum, chromium, iron or other transition metals in varying amounts. The catalysts can be in a massive form or they can be supported on a carrier such as the preferred silica or alumina carriers wherein the metal is present on the surface of the catalyst in a polyatomic form. Preferred catalysts are Raney nickel or Raney cobalt or a Ni/Re/B on silica catalyst prepared as described in U.S. Patent No. 4,123,462. The catalyst charge, as a weight percent of the total charge, is not narrowly critical, although a charge of about 3 weight percent is preferred for the reaction temperature and times taught in U.S. Ser. No. 454,485. The reaction conditions and catalysts of U.S. Ser. No. 454,485 can be used in the reaction zone of the invention.

The pertinent portions of copending, commonly-assigned U.S. Patent Application Serial No. 613,116, filed on May 23, 1984, are incorporated herein by reference. U.S. Ser. No. 613,116 discloses reductive amination catalysts which are catalysts comprising a support material selected from the group consisting of alumina, silica, silica-alumina, kieselguhr, diatomaceous earth and silica-titania, and nickel and at least one potentiating agent selected from the group consisting of platinum and iridium wherein the catalyst has a total nickel and potentiating agent content of about 1 to 30 percent by weight of the support and the atom ratio of the nickel to potentiating agent is in the range from about 1:1 to about 30:1. The potentiated nickel catalysts include catalysts which contain various other metals in admixture with the nickel and potentiating agent which do not detrimentally affect catalytic properties. These additional metals, in certain amination processes, may actually improve selectivity and activity of the potentiated nickel catalyst. Certain of these metals my extend the activity life and other physical properties of the catalyst. Examples of additional metal components include lanthanum, boron, magnesium, lithium, potassium, cesium, cerium, iron, ruthenium, copper, silver, zinc, cobalt, palladium, titanium, manganese, rhodium, and rhenium. The amount of such additional metal components, based on nickel and expressed as an atomic ratio, is about 0.001:1 to 1:1, frequently about 0.01:1 to 0.5:1. Particularly preferred catalysts comprise nickel, iridium and/or rhenium. In these catalysts the rhenium or iridium is generally provided in an atomic ratio to nickel of about 10:1 to 1:10.

When a reductive amination catalyst is used, the reaction temperature preferably is 150° to 235°C. and most preferably the reaction pressure is 200 to 2,000 p.s.i.g.

Any phosphorus-containing catalyst can be used in the second reactor. One group of preferred phosphorus-containing catalysts is the metal phosphate catalysts, which can be metal phosphates, metal monohydrogen phosphates, metal dihydrogen phosphates and metal pyrophosphates (although the latter is normally avoided in the invention process).

The metal phosphate catalysts include boron phosphate, aluminum phosphate, ferric phosphate, zinc phosphate, ferrous phosphate, nickel phosphate, chromium phosphate, copper phosphate and cobalt phosphate. Other metal phosphate catalysts which can be used are the phosphates of lithium, sodium, potassium, other metals of Group IA of the periodic tables, beryllium, magnesium, calcium, other metals of Group IIA of the periodic tables, titanium, zirconium, other metals of Group IVB of the periodic table, antimony and tin (valence states II and IV). Further useful catalysts are those which comprise a phosphorus bonded to a Group IVB transition metal oxide support, such as are disclosed in published European Patent Application 0115138 (the pertinent parts of which are incorporated herein by reference). Mixtures of two or more of the metal phosphate catalysts can be used.

The metal phosphate catalysts also include the pyrophosphates, monohydrogen phosphates and dihydrogen phosphates of strontium, copper, magnesium, calcium, barium, zinc, aluminum, cobalt, nickel, cerium, neodymium, and mixtures thereof. Specific examples of such catalysts are $SrHPO_4$, $Sr/BaHPO_4$, $Sr(H_2PO_4)_2$, $Ca(H_2PO_4)_2$, $Nd_2(HPO_4)_3$, $Ce_2(HPO_4)_3$, $CoHPO_4$, $NiHPO_4$, $Al_2(HPO_4)_3$, $MgHPO_4$, $BaHPO_4$, $CuHPO_4$ and $ZnHPO_4$.

The metal phosphate catalysts include the crystalline zirconium phosphates of U. S. Patent No. 3,416,884 and the granular zirconium phosphates of U. S. Patent No. 4,025,608 -the pertinent parts of such patents are incorporated herein by reference.

The metal phosphate catalysts which are most preferred for practicing the process of the invention are Group IIIB metal acid phosphates including Group IIIB metal phosphates, monohydrogen phosphates, dihydrogen phosphates and mixtures thereof. U. S. Patent No. 4,463,193 (the pertinent parts of the patent are incorporated herein by reference) discloses processes for preparing the Group IIIB metal acid phosphates. While the intent of the catalyst preparation is to specifically provide a particular Group IIIB monohydrogen phosphate or dihydrogen phosphate, mixtures of the Group IIIB metal phosphates of the above-mentioned types may be obtained owing to complicated dependence of the catalyst composition on preparation

conditions. Nevertheless, although the Group IIIB metal acid phosphate catalyst of the invention comprises the metal phosphate, monohydrogen phosphate, dihydrogen phosphate or mixtures thereof, the monohydrogen and dihydrogen phosphates of the Group IIIB metals are the preferred catalysts when in relatively pure form individually or in combination.

A Group IIIB metal is meant to include scandium, yttrium, lanthanum and the rare earth lanthanide metals having atomic numbers 58 to 71, and the rare earth actinides having atomic numbers 89 to 92.

The most preferred metal phosphate catalysts for the production of noncyclic polyalkylene polyamines are the acid phosphates, preferably the monohydrogen phosphates and dihydrogen phosphates, of scandium, lanthanum, cerium, samarium, europium, thulium, erbium, ytterbium, yttrium, lutetium, thorium, neodymium, praseodymium, dysprosium and gadolinium.

The acid phosphate catalysts can be used for the production of polyalkylene either singly or in combination.

It is preferred to use those which are more catalytically active and provide for substantial conversion to the noncyclic polyalkylene polyamine products. Examples of the most preferred catalyst compounds include lanthanum monohydrogen phosphate, lanthanum dihydrogen phosphate, lanthanum phosphate, praseodymium monohydrogen phosphate, praseodymium dihydrogen phosphate, praseodymium phosphate, neodymium monohydrogen phosphate, neodymium dihydrogen phosphate, neodymium phosphate and mixtures thereof.

The quantity of the acid phosphate salts of the Group IIIB metals used in the reaction can vary widely depending upon the reactivity of the catalysts and the reactivity of the reactants present. A catalytically effective amount of material is used; in other words, an amount which causes a reaction involving ammonia or an amine, the alkyleneamine and the alkanolamine to yield noncyclic polyalkylene products at the temperature and pressure used. Usually though, the amount used to provide a catalytic effect ranges from about 0.1 to 25 mole percent based upon the total amount of alkyleneamine and alkanolamine feed present in the reaction mixture, and preferably is an amount of about 0.1 to 10 mole percent. Within these ranges though, the level of catalyst is empirical and is adjusted depending on the product slate desired.

The Group IIIB metal phosphate catalysts used in the process of the invention can be prepared by the precipitation of the desired metal acid phosphate salt, washing the salt to remove inorganic coproducts and drying the salt. Optionally, dried catalysts can be further processed prior to use for polyalkylene polyamine manufacture.

Such process is well known to those skilled in the art and includes extrusion or pelletizing or compounding with an inert support such as alpha-alumina.

Methods of preparing Group IIIB metal monohydrogen phosphate or dihydrogen phosphate are disclosed in U. S. Patent No. 4,324,917 (the pertinent parts of the patent are incorporated herein by reference). Phosphate-containing materials can be obtained which consist predominantly of the Group IIIB metal phosphate, the Group IIIB metal monohydrogen phosphate, the Group IIIB metal dihydrogen phosphate, or mixtures in varying proportions of the Group IIIB metal monohydrogen and dihydrogen phosphate, and/or mixtures in varying proportions of any of the above the Group IIIB metal monohydrogen and dihydrogen phosphates with the Group IIIB metal phosphate. Such variations in catalyst composition can result from dependence of the catalyst composition on preparation conditions, such as temperature, concentration of reagents, stoichiometry of reagents, rate and order of reagent additions, pH of preparation, duration of preparation, volume and pH of water wash, duration of catalyst washing, and duration and temperature of catalyst drying. In any event, the Group IIIB metal acid phosphates obtained according to the general preparations referred to above are catalytically active for the production of polyalkylene polyamines.

Published European Patent Application 0115138 (the pertinent parts of which are incorporated herein by reference) discloses methods for preparing the catalysts comprising a phosphrus bonded to a Group IVB metal oxide support. Any appropriate liquid or liquefiable phosphorus compound can be used as a source of the phosphorus. For convenience, phosphoric acid will normally be used. However, other phosphorus compounds such as phosphoryl chloride ($POCl_3$), phosphorus acid, polyphosphoric acid, phosphorus halides, such as phosphorus bromide, alkyl phosphates and alkyl phosphites such as trimethyl phosphate, triethyl phosphate, trimethyl phosphite, triethyl phosphite, etc. may be utilized. Also, a diaminohydrogen phosphate such as diammonium hydrogen phosphate, $(NH_4)_2HPO_4$, dimethyldiamino hydrogen phosphate, $(CH_3)_2NH\ PO_4$, diethylaminohydrogen phosphate $(CH_3CH_2)_2NH\ PO_4$, etc. may be used.

The catalyst compositions are prepared by depositing a phosphorus compound on a support comprising an oxide of a group IVb transition metal oxide. The group IVb metal oxides include the oxides of titanium, zirconium, hafnium and thorium. Pellets of the group IVb metal oxide may be prepared by extrusion or by compaction in conventional pelleting apparatus using a pelleting aid such as graphite. The phosphorus compound can be on a powdered IVb metal oxide followed by pelleting and calcination. Preferably the catalyst composition is prepared by impregnating a preformed pellet. A suitable procedure to be used is to heat a liquid containing the liquid or liquefiable phosphorus compound at a temperature of about 100° to about 150°C. and to then add pellets in an amount equal to the volume of the heated liquid. This treatment should be continued from about 0.5 to about 5 hours. At the end of that time, the resulting mixture of pellets and liquid is adequate to substantially completely remove unadsorbed liquid. Temperatures above 150°C. can be used, if desired, but there is no particular advantage in doing so. It will be understood that the phosphorus that is present on a thus-treated pellet is not present as elemental phosphorus, but rather as phosphorus that is chemically bound, probably as an oxide, to the group IVb metal oxide support. However, the exact nature of the bonding is not completely understood.

European Patent Application 0115138 discloses the amount of phosphorus that is bonded or otherwise adheres to the support in a function of heating and other conditions used in the treating step and is also a function of the chemical identity of the phosphorus compound that is used as a source of phosphorus. Under the treating conditions exemplified above, at least about 2.5 weight percent of phosphorus is caused to bond or otherwise permanently adhere to the pellets. There is an upper limit to the amout of phosphorus that bonds or otherwise permanently adheres to the support. This upper limit is, as indicated, a function of both the treating conditions and the chemical used as a source of the phosphorus. Normally, the maximum amount of phosphorus that can be caused to bond or otherwise permanently adhere to the pellets is within the range of about 5 to 10 weight percent. As a matter of convenience, the normal practice is to use only one chemical as a phosphorus source - (e.g., phosphoric acid). However, mixtures of two or more such reagents may be used, if desired. Calcining is not mandatory if the pellets are impregnated at least at about 100°C., but the pellets can be calcined, if desired. Calcining is conducted for 2 to 24 hours at a temperature of from 100°C to below the temperature at which thermal destruction of the phosphorus bonding occurs. This can be determined experimentally for a particular catalyst. Temperatures above 900°C. should be avoided. A suitable calcining temperature range is normally 200° to 800°C. and, more preferably 500° to 700°C. Other procedures can be used in adding phosphorus to the group IVb metal oxide.

The pertinent parts of copending, commonly-assigned U.S. Application Ser. No. 576,807, filed on February 7, 1984, are incorporated herein by reference. U.S. Ser. No. 576,807 discloses certain phosphorus acid or acid derivative compounds which are useful phosphorus-containing catalysts within the scope of the invention herein. The term phosphorus acid or acid derivative defines compounds having a P-X bond wherein P is a phosphorus atom bonded to a halogen, oxygen, sulfur or nitrogen atom and wherein X which is a radical capable of (1) hydrolyzing to produce the corresponding phosphorus acid structure, or (2) exchanging with a hydroxyl group from the hydroxy alkylene reactant to provide a phosphorus ester.

The phosphorus acid or acid derivative catalyst of U. S. Ser. No. 576,807 is believed to function by forming with the alkanolamine or alkylene glycol compound a phosphorus ester in situ. For this reason, it is believed that a requirement for a good phosphorus catalyst is that it contain a substructure an atom bonded to phosphorus that can be replaced readily by the oxygen atom of a hydroxyl group of the difunctional hydroxy alkylene compound. Such a replaceable atom might be oxygen - (as in the case of phosphorous or phosphoric acid or their esters), halogen, nitrogen (as in the case of amides of phosphorous or phosphoric acids) or another atom that can be transformed into a phosphorus ester by a similar process.

Phosphorus-containing compounds such as trialkyl and triaryl phosphines and phosphine oxides, which contain no such exchangeable substructure, do not function as catalysts as defined in U. S. Ser. No. 576,807. Very sterically hindered phosphorus compounds such as hexaethyl phosphoric triamide, while containing the requisite exchangeable substructure and functioning to some extent, are less preferred catalysts because they undergo the exchange process with the alkanolamine or alkylene glycol hydroxyl moieties only slowly. Phosphorus acids are defined by those structures wherein X in the P-X radical is a hydroxyl radical. Acid derivatives are defined by structures wherein X is a substitute functional group. Various acids derivatives include: salts when -X is $-O^-M^+$ wherein $M^+$ is a mono or polyvalent cation; amides when -X is bonded to the phosphorus atom through a nitrogen atom; anhydrides when -X contains a second phosphorus atom bonded to the first phosphorus atom through an oxygen atom; esters when -X is -OR; and so on with regard to other functional groups defined by -X. The precise phosphorus acid or acid derivative structure is not critical so long as it fulfills the following two functional requirements: (1) that it provides for the relatively selective production of predominantly linearly extended polyalkylene polyamines and (2) that it enables increased conversion rates for polyalkylene polyamine production when water is removed during the reaction, possibly due to the water-inhibited formation of a phosphorus intermediate compound during the reaction.

The phosphorus acids or acid derivative catalysts of U. S. Ser. No. 576,807 include those having the structure:

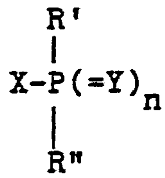

wherein Y is an oxygen or sulfur atom; n is 0 or 1, X is hydroxy, alkoxy, aryloxy, or the thio analogs of the foregoing, alkyl or aryl substituted amino, halo, or the salts or phosphorus anhydrides or thioanhydrides of the foregoing when X is hydroxy or mercapto; R' and R" are hydrogen, alkyl, aryl or one of the groups previously defined by X.

Suitable phosphorus acid or acid derivatives of U.S. Ser. No. 576,807 which can be employed include, for example, acidic metal or semi-metal phosphates, phosphoric acid compounds, and their anhydrides, phosphorous acid compounds and anhydrides, alkyl or aryl phosphates, alkyl or aryl phosphites, alkyl or aryl substituted phosphonic acids and phosphinic acids, alkali metal monosalts or phosphoric acid, phosphorous amides and phosphoric amides, the thioanalogs of the foreging, and mixtures of any of the above. Suitable acidic metal or semi-metal phosphates include boron phosphate, ferric phosphate, aluminum phosphate and the like. Suitable phosphoric acid compounds include aqueous or anhydrous phosphoric acids, such as orthophosphoric acid, pyrophosphoric acid, metaphosphoric acid, and condensed phosphoric acids such as polyphosphoric acids. Any commercially available mono-, di-, or trialkyl or aryl phosphate or phosphate ester can be employed. In addition, bis-(phosphates) and secondary phosphate esters, such as those disclosed in U. S. Patent No. 3,869,526 and U. S. Patent No. 3,869,527, respectively, can be utilized. Suitable alkyl or aryl substituted phosphonic acids or phosphinic acids include alkyl phosphonic acids, aryl phosphonic acids, alkyl phosphinic acids and aryl phosphinic acids. Examples of such phosphorus acid or acid derivative compounds include phenylphosphinic, ethylphosphinic, phenylphosphonic, naphthaphosphonic, and methylphosphinic acids; methyl phenylphosphonate, dimethyl phenylphosphonate, methyl phenyphosphinate, ethyl naphthaphosphinate, propyl methylphosphonate; hexamethyl phosphoric triamide, hexaethyl phosphoric triamide and their analogous phosphorous triamides. Preferred phosphorus catalysts include hexamethyl phosphorous triamide, hexaethyl phosphorous triamide, boron phosphate, ferric phosphate, aluminum phosphate, phosphoric acid and phosphorous acid.

The amount of phosphorus acid or acid derivative catalyst of U. S. Ser. No. 576,807 utilized is a catalytically effective amount to cause condensation of the reactants to produce predominantly diethylenetriamine. This quantity will vary depending upon the reaction conditions and catalyst utilized. Usually a catalytically effective amount will be from about 0.01 to about 10 mole percent and preferably from about 1 to about 3 mole percent, based on the moles of hydroxy alkylene compound used.

The pertinent parts of copending, commonly-assigned U. S. Patent Application Ser. No. 606,000, filed on May 2, 1984, are incorporated herein by reference. U. S. Ser. No. 606,000 discloses certain phosphorus amide catalysts which are compounds having at least one phosphorus-nitrogen, i.e., P-N, bond. Preferably, the P-N bond is part of a P-N-H or P-N-C substructure. Compounds containing suitable P-N bonds can have three, four, or five substituents about the phosphorus.

Suitable compounds catalysts of U. S. Ser. No. 606,000 having three substituents about phosphorus can be defined by the formula:

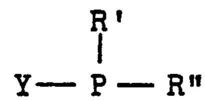

wherein Y is an unsubstituted or alkyl and/or aryl substituted amino radical; R' and R" are hydroxy, alkoxy, arayloxy, or their thio analogs, hydrogen, alkyl, aryl, halo, or one of the groups previously defined by Y, and can be joined together with each other or with Y to form a phosphorus-containing heterocyclic ring. If R', R", or Y contains hydrogen bonded to O, S, or N, such as when R' or R" is hydroxy or mercapto or Y is monoalkylamino, then corresponding metal salts containing P-O-M, P-S-M, or P-N-M linkages, where M is a monovalent or polyvalent metal or semimetal ion, and anhydrides, thioanhydrides, and condensed phosphorus amides containing respectively P-O-P, P-S-P, and P-N-P linkages can be suitable catalysts as well.

Suitable phosphorus amide catalysts of U. S. Ser. No. 606,000 having four substituents about phosphorus include those having the formula:

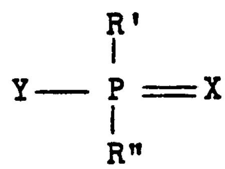

wherein X is an oxygen or sulfur atom, preferably oxygen, and Y, R', and R" are as defined above. As previously, corresponding metal and semimetal salts and condensed phosphorus compounds may also be suitable.

Suitable phosphorus amide catalysts of U. S. Er. No. 606,000 having five substituents about phosphorus include those having the formula:

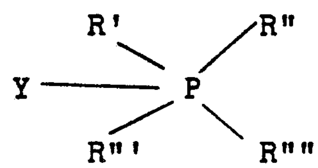

wherein Y is defined as above and R', R", R" ', and R"" are as defined for R' and R" above. As previously, corresponding metal and semimetal salts and condensed phosphorus compounds may also be suitable.

Suitable phosphorus amide compounds which can be employed include, for example, the following compounds or their alkyl or aryl derivatives:

| | |
|---|---|
| phosphoramidous acid, | $H_2N-P(OH)_2$; |
| phosphordiamidous acid, | $(H_2N)_2POH$; |
| phosphordiamidic acid, | $(N_2N)_2P(O)(OH)$; |
| phosphoramidic acid, | $H_2NP(O)(OH)_2$; |
| alkyl and aryl phosphonamidic acids, | $RP(O)(OH)NH_2$; |
| alkyl and aryl phosphonamidous acids, | $RP(OH)NH_2$; |
| esters and half-esters of the foregoing, | e.g. $H_2NP(OEt)_2$; |
| metal salts of the foregoing, | e.g. $H_2NP(O)_2K_2$; |
| triaminophosphine, | $(H_2N)_3P$; |
| triaminophosphine oxide, | $(H_2N)_3P(O)$; |
| alkyl and aryl phosphonic diamides, | $RP(O)(NH_2)_2$; |
| alkyl and aryl phosphonous diamides, | $RP(NH_2)_2$; |
| alkyl and aryl phosphinous amides, | $R_2P(NH_2)$; |
| alkyl and aryl phosphinic amides, | $R_2P(O)(NH_2)$; |

analogs of the foregoing substituted with alkyl or aryl groups on nitrogen, e.g. $R_2NP(OH)_2$; and thioanalogs of the foregoing, e.g. $R_2(NP(S)OEt)_2$.

The alkyl or aryl substituents on these substances can be linked to phosphorus through more than one atom, so as to form cyclic members of the above classes containing such heterocyclic rings as

1,3,2-diazaphospholidine,

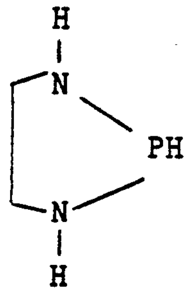

; 1,3,2,-oxazaphospholidine,

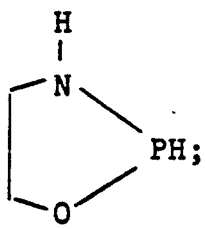

tetrahydro-2H-1,3-2-oxazaphosphorine,

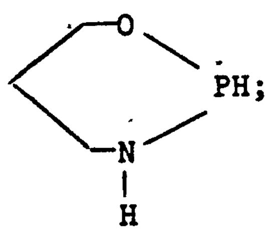

and the like. Such cyclic phosphorous amides can also be used as catalysts in the invention.

An additional class of phosphorus amides of U. S. Ser. No. 606,000 that can be useful as catalysts in the invention comprise azophosphorances in which nitrogen is bound directly to phosphorus. Examples of such compounds include: 1,6-dioxa-4,9-diaza-5-phospha-(5-$P^V$) spiro [4.4]-nonane,

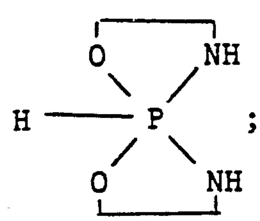

and

2,3,5,6,8,8-hexahydro-8-methyl-[1,3,2]-oxazaphospholo-[2,3-b][1,3,2]oxazaphosphole,

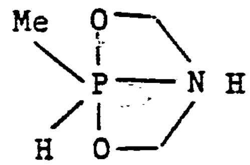

Preferred phosphorus amide catalysts of U. S. Ser. No. 606,000 include hexamethyl phosphorous triamide, hexaethyl phosphorous triamide and the phosphorus amide reaction product of ethylenediamine with phosphoric or phosphorous acid.

Phosphorus-containing cation exchange resins can be used in this invention and can be prepared by the methods disclosed in U.S. Patent No. 4,324,917. The cation exchange resins provide exchangeable phosphorus-containing ions such as phosphonous, phosphonic, phosphoric and phosphorus. Preferably the resins useful here are weak-acid cation exchange resins containing one or more of the above phosphorus-containing exchangeable ions. Duolite® resins available from Diamond Shamrock Corp. are typical commercial phosphorus-containing resins. Examples of these are Duolite® ES-62, Duolite® ES-63, and Duolite® ES-65 which are the phosphonous, phosphonic and phosphoric acid types, respectively.

When a phosphorus-containing catalyst is used, the reaction temperature preferably is 220° to 350°C. (most preferably 260° to 300°C.) and most preferably the reaction pressure is 200 to 2,000 p.s.i.g.

Any of the non-resin catalysts useful in the invention can be supported on carriers, such as, silica, silica-alumina, silica-titania, alumina, diatomaceous earth (Kieselguhr) and any other conventionally-employed reactor packing material. Generally, the catalysts are supported. The active catalyst species are provided on the surface of the support through, for example, coating or impregnation. The catalyst (say metal) components on the support often comprise about 1 to 50, say, about 3 to 30, weight percent of the catalyst. useful supports can be porous and have surface areas of from about 0.1 to 500, say, about 0.3 to 100, square meters per gram.

The catalyst can be of any convenient size or shape. Catalysts can be made in the form of powders, spherical or conical pellets, extruded strips and the like. Impregnated spherical pellets ranging in diameter from 1/8 inch to 3/16 inch and extruded strips of a cylindrical-type shape ranging from 1/32 inch to 1/2 inch in length are typical of those which can be used as supports. Often, for commerical-scale operations, the pellets range in diameter from about 0.1 to 1 centimeter.

One of the purposes of the second catalyst in the series is to allow use of a crude feed containing alkylenediamine produced in the first reactor

Recovery of the polyaklylene polyamines, alkanolamine and alkylenediamine from the reaction mixture from the second reactor can be accomplished by conventional techniques.

Preferably the separation step is conducted using distillation. Most preferably the separation is conducted using a fractional distillation column with the polyethylene polyamines coming off of the bottom of the column and with the unreacted alkanolamine and alkylenediamine coming off the top portion of the column. With a fractional distillation column, for example, having 10 trays, a pressure of 1000 p.s.i.g. and a base temperature of 220°C., 90 mole percent of the unreacted ethanolamine and ethylenediamine come off of the top of the column with the unreacted $NH_3$ and $H_2$ and 90 mole percent of the diethylenetriamine (and most of the AEEA and piperazine) comes off of the bottom of the column.

The separation can be conducted in adsorbers, with the adsorbing liquids removing the polyalkylene polyamines from unreacted alkanolamine and alkylenediamine. Preferably the adsorbing liquid for the polyalkylene polyamines is triethylenetetramine or a higher boiling adsorbent.

The separation can also be conducted using a series of partial condensers. The alkanolamine and alkylenediamine condense out in the last of the partial condensers, and the polyalkylene polyamines condensing out in the first of the partial condensers. Preferably 3 to 5 partial condensers are used.

The recycle of the alkylenediamine and alkanolamine, as explained above, allow control of the molecular weight range and distribution of the polyalkylene polyamine product.

Generally, the mole ratio of alkyleneamine compound to alkanolamine compound can range from about 0.05:1 to 12:1, and preferably is about 0.75:1 to 10:1. It is preferred when reacting

ethylenediamine (EDA) and monoethanolamine - (MEA) with ammonia in the reactor that the mole ratios be in a range of 0.05-10:1:0.35-20 (EDA:MEA:$NH_3$).

The analysis of the products produced by the process of the invention can be conducted by using standard gas chromatography techniques using columns selected from their ability to separate the individual components that may be present in a particular reaction mixture.

Polyethylene polyamines are useful as corrosion inhibitors, fabric softeners, lubricating oil additives, co-monomers for polyamide resins, fungicides, surfactants, curing agents for epoxy resins and chelating agents.

In the embodiment of the invention which is a process for the production of higher molecular weight polyalkylene polyamines from a feed which includes oxygen-containing higher molecular weight polyalkylene polyamines, the oxygen-containing higher molecular weight polyalkylenes polyamines are contacted with an alkyleneamine having at least two amino groups in the presence of a catalytically effective amount of an amination catalyst, such as, a reductive amination catalyst or a phosphorus-containing catalyst, at a temperature and pressure effective to conduct such reaction. A gaseous diluent or ammonia can be added to the reactor. Preferably the reaction is conducted in the vapor phase or supercritical phase. Preferably the produced higher molecular weight polyalkylene polyamines have at least four amino groups. Also preferably the oxygen-containing higher molecular weight polyalkylene polyamines are hydroxyl-containing higher molecular weight polyethylene polyamines.

The oxygen-containing higher molecular weight polyethylene polyamines feed can be at least part the bottoms portions obtained from a distillation column for separating diethylenetriamine from said bottoms portion. The reaction mixture from the reaction step can be recycled at least in part to the reaction zone and/or at least part thereof can be separated using a batch still into a stream containing the higher molecular weight polyethylene polyamines and a stream containing water and ethylenediamine. Preferably at least part of the stream containing water and ethylenediamine is recycled to the reaction step.

In Figure 1, MEA, ammonia, EDA, AEEA and sometimes DETA and higher polyethylene polyamines are fed into reactor 10 via line 12. Preferably a liquid hourly space velocity of the reactants of 1 to 25 $hr^{-1}$ is used. A Group IIIB acid phosphate catalyst or a Ni-B-Re catalyst is preferably used in reactor 10. Reactor 10 is preferably run at a temperature of 220° to 350°. for the Group IIIB metal phosphate catalyst or 150° to 235°C. for the Ni-B-Re catalyst, and at a pressure of 200 to 2,000 p.s.i.g. The reaction stream exiting reactor 10 via line 14 contains DETA, higher polyethylene polyamines, and the unreacted EDA, MEA and ammonia. If hydrogen or diluent is used in the feed, the reaction stream will also contain such materials. The DETA and higher polyethylene polyamines can be separated using any conventional or suitable separation means. Preferably the separation is achieved using a fractional distillation column.

By controlling the relative amounts of monoalkanolamine, ammonia, aminoethylethanolamine and/or ethylenediamine in the feed to reactor 10, the molecular range and distribution of the polyalkylene polyamines obtained from reactor 10 can be thereby affected as desired.

In Figure 2, MEA and ammonia are fed into first reactor 16 via line 18. Preferably a liquid space velocity of the reactants of .1 to 25 $hr^{-1}$ is used in first reactor 16. A Ni-B-Re catalyst is preferably used in first reactor 16. Preferably hydrogen is also present in the feed to first reactor 16. First reactor 16 is preferably run at a temperature of 150° to 235°C. and at a pressure of 200 to 2,000 p.s.i.g. A large excess of ammonia is preferably used in first reactor 16. MEA, ammonia, EDA, AEEA, some DETA and some higher polyethylene polyamines exit first reactor 16 via line 12 and are fed into reactor 10.

Preferably a space velocity of reactants of 1 to 20 $hr^{-1}$ is used in second reactor 10. A group IIIB acid phosphate catalyst or a Ni-B-Re is preferably used in second reactor 10. Second reactor 10 is preferably run at a temperature of 220° to 350°C. for the Group IIIB metal phosphate catalyst or 150° to 235°C. for the Ni-B-Re catalyst, and at a pressure of 200 to 2,000 p.s.i.g. The reaction stream exiting second reactor 10 via line 14 contains DETA, higher polyethylene polyamines, and the unreacted EDA, MEA and ammonia. If hydrogen or a diluent is used in the feed to first reactor 16, the reaction stream from second reactor 10 will also contain such materials. The DETA and higher polyethylene polyamines can be separated using any conventional or suitable separation means. Preferably the separation is achieved using a fractional distillation column.

By controlling the relative amounts of monoalkanolamine and amine in the feed to first reactor 16, the molecular range and distribution of the polyalkylene polyamines obtained from reactor 10 can be thereby affected as desired.

In Figure 3, ammonia is fed into first reaction 16 via lines 20 and 18. MEA is fed into reactor 16 via lines 24, 22 and 18. Preferably a liquid hourly space velocity of the reactants of 1 to 25 $hr^{-1}$ is used in first reactor 16. A Ni-B-Re catalyst is preferably used in first reactor 16. Preferably hydrogen is also present in the feed to first reactor 16. First reactor 16 is preferably run at a temperature of 150° to 250°C. and at a pressure of 200 to 2,000 p.s.i.g. A large excess of ammonia is preferably used in first reactor 16. MEA, ammonia, EDA, AEEA, some DETA and some higher polyethylene polyamines exit first reactor 16 via line 12 and are fed into reactor 10.

Preferably a liquid hourly space velocity of reactants of 1 to 25 $hr^{-1}$ is used in second reactor 10. A Group IIIB acid phosphate catalyst or a Ni-B-Re is preferably used in second reactor 10. Second reactor 10 is preferably run at a temperature of 220° to 350°C. for the Group IIIB metal phosphate catalyst or 150° to 235°C. for the Ni-B-Recatalyst and at a pressure of 500 to 1,500 p.s.i.g. The reaction stream exiting second reactor 10 via line 14 contains DETA, higher polyethylene polyamines, and the unreacted EDA, MEA and ammonia. If hydrogen or a diluent is used in the feed to first reactor 16, the reaction stream from second reactor 10 will also contain such materials.

The reaction feed exiting second reactor 10 is fed into separator 34, which is preferably a fractional distillation column. The DETA and the higher polyethylene polyamines are separated (line 26) out of the bottom of separator 34 from the other materials. At least a part of EDA (plus some MEA and water) is separated via line 28 from separator 34 and is fed into line 12 to thereby control or regulate the amount of EDA being fed into second reactor 10. In this manner the molecular range and distribution of the polyethylene polyamines obtained from reactor 10 can be thereby affected as desired. Some of the separated EDA (plus some MEA and $H_2O$) can be removed from the system via lines 28-36. The MEA-containing stream separated via line 38 from separator 40 is fed into lines 22 and 18 to thereby control or regulate the amount of MEA being fed into first reactor 16. In this manner the molecular range and distribution of polyethylene polyamines obtained from reactor 10 can be thereby affected as desired.

Using the reactor scheme of Figure 3, 1 mole of MEA and 15 moles of ammonia (plus 3 moles of hydrogen) aree fed into reactor 16, which is operated at 200°C. and 1,100 p.s.i.g. A nickel-rhenium-boron catalyst is used. When a fifty mole percent conversion is achieved in reactor 16, the reaction stream therefrom is sent to reactor 10. One mole of EDA is added to the reaction stream, which contains 0.5 mole of MEA, 1.5 mole of EDA and 15 moles of ammonia (plus 3 moles of hydrogen). After the cumulative conversion reaches 90 mole percent in reactor 10, the product stream therefrom is sent to separator 34, which is a fractional distillation column (10 trays; bottom temperature of 220°C.; and a pressure of 1000 p.s.i.g.). The product is DETA and higher polyethylene polyamines. Separated MEA (from separation zone 40) and separated EDA are recycled to the feed of reactor 16 as desired. The MEA and EDA recycle allows control of the molecular distribution and range of the polyethylene polyamine product.

Copending, commonly-assigned patent application entitled "Interreactor Separator", (D-14871), concurrently filed with this application, is incorporated herein by reference. Such application discloses the use of a reductive amination reaction zone from which its effluent is separated into a, preferably, gaseous, MEA-and EDA-containing phase and a liquid, DETA-rich phase. The gas phase can be used for recycle (advantageously, it is at high pressure and suitable for recycle without undue energy penalties) or as a feed to another reactor which can use a reductive amination or other type (e.g., phosphorus-based catalysts) of catalysts.

Copending, commonly-assigned patent application entitled "Improving the Quality of Catalytically Prepared Polyalkylene Polyamines", (D-14872), concurrently filed with this application, is incorporated herein by reference. Such application discloses processes for making polyalkylene polyamines from ethanolamines and a nitrogen compound (e.g., ammonia or alkyleneamine) using a phosphorus-based catalyst in the presence of sufficient hydrogen to enhance color and reduce odor.

Copending, commonly-assigned patent application entitled "Two Reactor Scheme Using Only Phosphorus-Containing Catalysts", (D-14874), concurrently filed with this application. Such application discloses passing an oxygenated polyethylenepolyamine feed in the presence of a nitrogen compound to a reaction zone containing a phosphorus-based catalyst. This feed is obtained from a polyethylenepolyamine reactor having a phosphorus-based catalyst. The feed typically contains AEEA.

Copending, commonly-assigned patent application entitled "Interchangeable Process Scheme", (D-14875), concurrently filed with this application, is incorporated herein by reference. Such application discloses processes for making and separating ethyleneamines, alkylamines, morpholine, etc., in

same process equipment. The separation systems required for these processes are very similar. Hence, block operation in the same reactor and separation equipment is achieved.

Copending, commonly-assigned patent application entitled "Preparation of Diethylenetriamine With Azeotrope Recycle", (D-14876), concurrently filed with this application, is incorporated herein by reference. Such application discloses processes for making ethyleneamines using a reductive amination or phosphorus-based catalyst in which a gaseous EDA/water phase is separated from the reactor effluent and at least a portion of the separated phase is returned to the reactor. In a preferred embodiment using a reductive amination catalyst, the recycle stream is admixed with MEA to break the azeotrope with water condensing out.

Copending, commonly-assigned patent application entitled "Water Addition to Enhance Phosphoric Acid Salt Catalyst Activity", (D-14877), concurrently filed with this application, is incorporated herein by reference. Such application discloses providing beneficial amounts of water in the reaction zone in which alkanolamine and another nitrogen compound are reacted to produce alkyleneamines over a phosphorus-based catalyst. The water is believed to enhance or maintain or regenerate catalytic activity.

Copending, commonly-assigned patent application entitled "Use of Pressure To Control the Noncyclics/Cyclics Ratio of Polyalkylene Polyamines", (D-14878), concurrently filed with this application, is incorporated herein by reference. Such application discloses processes for producing polyalkylene polyamines over phosphorus-based catalysts in which control of the ratio of noncyclic to cyclic products is achieved by controlling the level of the reaction pressure.

The following compound abbreviations are sometimes used herein:

EDA -ethylenediamine

MEA -monoethanolamine

PIP -piperazine

AEP -aminoethylpiperazine

DETA -diethylenetriamine

TETA(NC)-triethylenetetramine (noncyclic isomers)

TETA(C) -triethylenetetramine (cyclic isomers)

TEPA(NC)-tetraethylenepentamine (noncyclic isomers)

TEPA(C) -tetraethylenepentamine (cyclic isomers)

HVY(NC) -pentaethylenehexamine and higher oligomeric polyethylene amines (noncyclic isomers)

HVY(C) -pentaethylenehexamine and higher oligomeric polyethylene amines (cyclic isomers)

AEEA -aminoethylethanolamine.

**Claims**

1. Process for preparing polyalkylene polyamines from a feed composed of (i) an alkanolamine having at least one amino group, (ii) an alkyleneamine having at least two amino groups, (iii) an oxygenated organic amine compound other than said alkanolamine (i), and (iv) a reactive nitrogen-containing base selected from the group consisting of ammonia or a primary amine and a secondary amine, comprising contacting said alkanolamine, said alkyleneamine, said oxygenated organic amine compound and said reactive nitrogen-containing base in a reaction zone to produce said polyalkylene polyamines in the presence of a catalytically effective amount of an amination catalyst and at a temperature and pressure sufficient to form said polyalkylene polyamines.

2. The process as claimed in Claim 1 wherein said amination catalyst is a reductive amination catalyst.

3. The process as claimed in Claim 1 wherein said amination catalyst is a phosphorus-containing catalyst.

4. Process for preparing polyalkylene polyamines from a feed composed of (i) an alkanolamine, having at least one amino group, (ii) an alkyleneamine having at least two amino groups, (iii) aminoalkylalkanolamine and (iv) a reactive nitrogen-containing base selected from the group consisting of ammonia, a primary amine and a secondary amine, comprising contacting said alkanolamine, said alkyleneamine, said aminoalkylalkanolamine and said reactive nitrogen-containing base in a reaction zone to produce said polyalkylene polyamines in the presence of a catalytically effective amount of an amination catalyst and at a temperature and pressure sufficient to form said polyalkylene polyamines.

5. The process as claimed in Claim 4 wherein said alkanolamine is ethanolamine, said alkyleneamine is ethylenediamine, said aminoalkylalkanolamine is aminoethylethanolamine and said reactant (iv) is ammonia.

6. The process as claimed in Claim 4 wherein said reaction is conducted in the vapor phase or supercritical phase.

7. The process as claimed in Claim 4 wherein a gaseous diluent is added to said reaction.

8. The process as claimed in Claim 4 wherein said reaction is a continuous reaction.

9. The process as claimed in Claim 4 wherein said reaction is conducted at a pressure of about 200 to 2,000 psig.

10. The process as claimed in Claim 4 wherein the liquid hourly space velocity of said reactants in said reaction is between 0.1 and 100 per hour.

11. The process as claimed in Claim 4 wherein, in said reaction, when said amination catalyst is a reductive amination catalyst, the temperature is between 150° and 235°C.

12. The process as claimed in Claim 4 wherein, in said reaction, when said amination catalyst is a phosphorus-containing catalyst, the temperature is between 220° and 350°C.

13. The process as claimed in Claim 4 wherein said amination catalyst is a reductive amination catalyst.

14. The process as claimed in Claim 13 wherein said reductive amination catalyst is comprised of nickel impregnated on a conventional catalyst support material.

15. The process as claimed in Claim 14 wherein said reductive amination catalyst is comprised of nickel impregnated on a support material selected from the group consisting of alumina, silica, silica-alumina, diatomaceous earth, siliça-titania and mixtures of at least two members of such group.

16. The process as claimed in Claim 15 wherein a small percentage of rhenium and boron is present in said nickel.

17. The process as claimed in Claim 4 wherein said amination catalyst is a phosphorus-containing catalyst.

18. The process as claimed in Claim 17 wherein said phosphorus-containing catalyst is a phosphorus acid or a phosphorus acid derivative compound.

19. The process as claimed in Claim 17 wherein phosphorus-containing catalyst is a metal phosphate catalyst.

20. The process as claimed in Claim 17 wherein said metal phosphate catalyst is a metal acid phosphate catlayst.

21. The process as claimed in Claim 18 wherein said metal acid phosphate catalyst is a solid, insoluble, metal acid phosphate catalyst.

22. The process as claimed in Claim 19 wherein said metal acid phosphate is a Group IIIB metal acid phosphate, a Group IIIB metal monohydrogen phosphate or a Group IIIB dihydrogen phosphate, and wherein said Group IIIB metal is scandium, yttrium, lanthanum or a rare earth lanthanide having an atomic number from 58 to 71.

23. The process as claimed in Claim 20 wherein said metal acid phosphate catalyst is a Group IIA metal acid phosphate catalyst or a Group IVB metal acid phosphate catalyst.

24. Process for preparing polyalkylene polyamines comprising:

(a) contacting in a first reaction zone (i) an alkanolamine having at least one amino group and (ii) a reactive nitrogen-containing base selected from the group consisting of ammonia, a primary amine and a secondary amine, to produce an alkyleneamine having at least two amino groups, and an oxygenated organic amine compound other than said alkanolamine (i), in the presence of a catalytically effective amount of a reductive amination catalyst and at a temperature and pressure sufficient to form said alkyleneamine and said oxygenated organic amine compound; and

(b) contacting in another reaction zone at least a part of said reaction product stream (a) comprising said alkyleneamine, said oxygenated organic amine compound, said unreacted alkanolamine and said unreacted reactive nitrogen-containing base to produce said polyalkylene polyamines in the presence of a catalytically effective amount of an amination catalyst and at a temperature and pressure sufficient to form said polyalkylene polyamines.

25. The process as claimed in Claim 24 wherein an alkyleneamine having at least two amino groups is included in the feed to said reaction zone (a).

26. The process as claimed in Claim 24 wherein said amination catalyst in step (b) is a reductive amination catalyst.

27. The process as claimed in Claim 24 wherein said amination catalyst in step (b) is a phosphorus-containing catalyst.

28. Process for preparing polyalkylene polyamines comprisng:

(a) contacting in a first reaction zone (i) an alkanolamine having at least one amino group and (ii) a reactive nitrogen-base compound selected from the group consisting of ammonia, a primary amine and a secondary amine, to produce an alkyleneamine having at least two amino groups and an aminoalkylalkanolamine in the presence of a catalytically effective amount of a reductive amination catalyst and at a temperature and pressure sufficient to form said alkyleneamine and said aminoalkylalkanolamine; and

(b) contacting in another reaction zone at least a part of said reaction product stream (a) comprising said alkyleneamine, said aminoalkylalkanolamine, said unreacted alkanolamine and said unreacted reactive nitrogen-containing base to produce said polyalkylene polyamines in the presence of a catalytically effective amount of an amination catalyst and at a temperature and pressure sufficient to form said polyalkylene polyamines.

29. The process as claimed in Claim 28 wherein an alkyleneamine having at least two amino groups is included in the feed to said reaction zone (a).

30. The process as claimed in Claim 28 wherein said alkanolamine is ethanolamine, wherein said alkyleneamine is ethylenediamine and wherein ammonia is used.

31. The process as claimed in Claim 28 wherein said reaction (a) is conducted in the vapor phase or supercritical phase.

32. The process as claimed in Claim 28 wherein a gaseous diluent is added to said reaction (a).

33. The process as claimed in Claim 28 wherein said reactions (a) and (b) are continuous reactions.

34. The process as claimed in Claim 28 wherein said amination catalyst in reaction (a) is a reductive amination catalyst.

35. The process as claimed in Claim 34 wherein said reductive amination catalyst is comprised of nickel impregnated on a conventional catalyst support material.

36. The process as claimed in Claim 34 wherein said reductive amination catalyst is comprised of nickel impregnated on a support material selected from the group consisting of alumina, silica, silica-alumina, diatomaceous earth, silica-titania and mixtures of at least two members of such group.

37. The process as claimed in Claim 36 wherein a small percentage of rhenium and boron is present in said nickel.

38. The process as claimed in Claim 36 wherein said reaction (a) is conducted at a presure of about 200 to 2,000 psig.

39. The process as claimed in Claim 36 wherein the liquid hourly space velocity of said reactants in said reaction (a) is between 0.1 and 100 per hour.

40. The process as claimed in Claim 28 wherein, in said reaction (a) the temperature is between 150° and 235°C.

41. The process as claimed in Claim 28 wherein, in said second reaction (b), said alkylenediamine is ethylenediamine, said alkanolamine is ethanolamine, and said aminoalkylalkanolamine is aminoethylethanolamine and wherein ammonia is used.

42. The process as claimed in Claim 28 wherein the liquid hourly space velocity of said reactants in said reaction (b) is between 0.1 and 100 per hour.

43. The process as claimed in Claim 28 wherein said reaction (b) is conducted in the vapor phase or supercritical phase.

44. The process as claimed in Claim 28 wherein said reaction (b) is conducted at a pressure of about 200 to about 2,000 psig.

45. The process as claimed in Claim 28 wherein, in said reaction (b), when said amination catalyst is a reductive amination catalyst, the temperature is between 150° and 235°C.

46. The process as claimed in Claim 28 wherein, in said reaction (b), when said amination catalyst is a phosphorus-containing catalyst, the temperature is between 220° and 350°C.

47. The process as claimed in Claim 28 wherein said amination catalyst in said reaction (b) is a reductive amination catalyst.

48. The process as claimed in Claim 47 wherein said reductive amination catalyst is comprised of nickel impregnated on a conventional catalyst support material.

49. The process as claimed in claim 47 wherein said reductive amination catalyst is comprised of nickel impregnated on a support material selected from the group consisting of alumina, silica, silica-alumina, diatomaceous earth, silica-titania and mixtures of at least two members of such group.

50. The process as claimed in Claim 49 wherein a small percentage of rhenium and boron are present in said nickel.

51. The process as claimed in Claim 28 wherein said amination catalyst in said reaction (b) is a phosphorus-containing catalyst.

52. The process as claimed in Claim 51 wherein said phosphorus-containing catalyst is a phosphorus acid or a phosphorus acid derivative compound.

53. The process as claimed in Claim 51 wherein phosphorus-containing catalysts is a metal phosphate catalyst.

54. The process as claimed in Claim 53 wherein said metal phosphate catalyst is a metal acid phosphate catalyst.

55. The process as claimed in Claim 54 wherein said metal acid phosphate catalyst is a solid, insoluble, metal acid phosphate catalyst.

56. The process as claimed in Claim 55 wherein said metal acid phosphate is a Group IIIB metal acid phosphate, a Group IIIB metal monohydrogen phosphate or a Group IIIB dihydrogen phosphate, and wherein said Group IIIB metal is scandium,

yttrium, lanthanum or a rare earth lanthanide having an atomic number from 58 to 71.

57. The process as claimed in Claim 54 wherein said metal acid phosphate catalyst is a Group IIA metal acid phosphate catalyst or a Group IVB metal acid phosphate catalyst.

58. Process for affecting the molecular range and distribution of polyalkylene polyamines, comprising:

(a) contacting an alkyleneamine having at least two amino groups, an alkanolamine having at least one amino group, a reactive nitrogen-containing base selected from the group consisting of ammonia, a primary amine and a secondary amine, and a stream containing an oxygenated organic amine compound other than said alkanolamine, in a reactor in the presence of a catalytically effective amount of an amination catalyst and at a temperature and pressure sufficient to form said polyalkylene polyamines;

(b) separating at least a part of said alkyleneamine as a separate component, and/or at least a part of said alkanolamine as a separate component, and/or a separate feed containing at least a part of said oxygenated organic amine compound, and/or at least a part of said polyalkylene polyamines as a separate component from at least a part of said reaction stream (a); and

(c) recycling at least part of said separated alkanolamine to said reaction (a), thereby affecting the molecular range and distribution of said polyalkylene polyamines product in said reaction (a) as desired; and/or

(d) recycling at least part of said separated alkyleneamine to said reaction (a), thereby affecting the molecular range and distribution of said polyalkylene polyamines produced in said reaction (a) as desired; and/or

(e) recycling at least part of said separated stream containing said oxygenated organic amine compound to said reaction (a), thereby affecting the molecular range and distribution of said polyalkylene polyamines produced in said reaction (a) as desired.

59. The process as claimed in Claim 58 wherein an alkyleneamine having at least two amino groups is included in the feed to said reaction zone (a).

60. The process as claimed in Claim 58 wherein said amination catalyst in step (b) is a reductive amination catalyst.

61. The process as claimed in Claim 58 wherein said amination catalyst in step (b) is a phosphorus-containing catalyst.

62. Process for affecting the molecular range and distribution of polyalkylene polyamines, comprising:

(a) contacting an alkyleneamine having at least two amino groups, an alkanolamine having at least one amino group, a reactive nitrogen-containing base selected from the group consisting of ammonia, a primary amine and a secondary amine, and a stream containing aminoalkylalkanolamine, in a reactor in the presence of a catalytically effective amount of an amination catalyst and at a temperature and pressure sufficient to form said polyalkylene polyamines;

(b) separating at least part of said alkyleneamine as a separate component, at least part of said alkanolamine as a separate component, a separate feed containing at least part of the aminoalkylalkanolamine, and at least part of said polyalkylene polyamines as a separate component from. at least a part of said reaction stream (a); and

(c) recycling at least part of said separated alkanolamine to said reaction (a), thereby affecting the molecular range and distribution of said polyalkylene polyamines product in said reaction (a) as desired; and/or

(d) recycling at least part of said separated alkyleneamine to said reaction (a), thereby affecting the molecular range and distribution of said polyalkylene polyamines produced in said reaction (a) as desired; and/or

(e) recycling at least part of said separated stream containing aminoalkylalkanolamine to said reaction (a) thereby affecting the molecular range and distribution of said polyalkylene polyamines produced in said reaction (a) as desired.

63. The process as claimed in Claim 62 wherein an alkyleneamine having at least two amino groups is included in the feed to said reaction zone (a).

64. The process as claimed in Claim 62 wherein said amination catalyst in step (b) is a reductive amination catalyst.

65. The process as claimed in Claim 62 wherein said amination catalyst in step (b) is a phosphorus-containing catalyst.

66. Process for affecting the molecular range and distribution of polyalkylene polyamines prepared from a feed composed of an alkanolamine having at least one amine group and a reactive nitrogen-containing base selected from the group consisting of ammonia or a primary amine or a secondary amine, comprising:

(a) contacting in a first reaction zone said reactive alkanolamine and said nitrogen-containing base to produce an alkyleneamine having at least two amino groups and an aminoalkylalkanolamine in the presence of a catalytically effective amount of a reductive amination catalyst and at a temperature and pressure sufficient to form said alkyleneamine and said aminoalkylalkanolamine;

(b) contacting in another reaction zone said alkyleneamine, said aminoalkylalkanolamine, said unreacted alkanolamine and said unreacted reactive nitrogen-containing base to produce said polyalkylene polyamines in the presence of a catalytically effective amount of an amination catalyst and at a temperature and pressure sufficient to form said polyalkylene polyamines;

(c) recycling at least part of said reaction stream (b) to said reaction (a) and/or said reaction (b); and/or

(d) separating at least a part of said alkylenamine as a separate component, at least a part of said alkanolamine as a separate component and at least a part of said polyalkylene polyamines as a separate component from at least a part of said reaction stream (b); and

(e) recycling at least part of said separated alkanolamine to said reaction (a), thereby affecting the molecular range and distribution of said polyalkylene polyamines product in said reaction (b) as desired; and/or

(f) recycling at least part of said separated alkyleneamine to said reaction (b), thereby affecting the molecular range and distribution of said polyalkylene polyamines produced in said reaction (b) as desired.

67. The process as claimed in Claim 66 wherein an alkyleneamine having at least two amino groups is included in the feed to said reaction zone (a).

68. The process as claimed in Claim 66 wherein at least a part of said separate alkanolamine to said reaction (a), thereby affecting the molecular range and distribution of said polyalkylene polyamines product in said reaction (b) as desired.

69. The process as claimed in Claim 66 wherein at least part of said separated alkyleneamine to said reaction (b), thereby affecting the molecular range and distribution of said polyalkylene polyamines produced in said reaction (b) as desired.

70. The process as claimed in Claim 66 wherein at least a part of said separated alkanolamine to said reaction (a) and at least part of said separated alkylenamine to said reaction (b), thereby affecting the molecular range and distribution of said polyalkylene polyamines product in said reaction (b) as desired.

71. The process as claimed in Claim 66 wherein said separation (d) is conducted using distillation.

72. The process as claimed in Claim 66 wherein said separation (i) is conducted using a fractional distillation column with said polyalkylene polyamines coming off of the bottom of said column and with said alkanolamine and said alkyleneamine coming off of the top portion of said column as separate components.

73. The process as claimed in Claim 66 wherein said reaction (a) is conducted in the vapor phase or supercritical phase.

74. The process as claimed in Claim 66 wherein said reaction (a) is conducted at a pressure of about 200 to 2,000 psig.

75. The process as claimed in Claim 66 wherein the liquid hourly space velocity of said reactants in said reaction (a) is between 0.1 and 100 per hour of said reactants.

76. The process as claimed in Claim 66 wherein, in said reaction (a) the temperature is between 150° and 235°C.

77. The process as claimed in Claim 66 wherein the liquid hourly space velocity of said reactants in said reaction (b) is between 0.1 to 100 per hour of reactants.

78. The process as claimed in Claim 66 wherein, in said reaction (b), said amination catalyst is a said phosphorus-containing catalyst.

79. The process as claimed in Claim 78 wherein the reaction temperature is between 220° and 350°C.

80. The process as claimed in Claim 66 wherein said reaction (b) is conducted in the vapor phase or supercritical phase.

81. The process as claimed in Claim 66 wherein, in said reaction (b), said amination the catalyst is a reductive amination catalyst.

82. The process as claimed in Claim 81 wherein the reaction temperature is between 150° and 235°C.

83. The process as claimed in Claim 66 wherein reaction (b) is conducted at a pressure of 200 to 2,000 psig.

84. Process for obtaining a specified range and distribution of polyalkylene polyamines prepared from a feed composed of (i) an alkanolamine having at least one amino group, (ii) an alkyleneamine having at least two amino groups, (iii) an aminoalkylalkanolamine and (iv) a reactive nitrogen-containing base selected from the group consisting of ammonia, a primary amine and a secondary amine, comprising:

(a) contacting said alkanolamine, said alkyleneamine, said aminoalkylalkanolamine and said reactive nitrogen-containing base to produce said polyalkylene polyamines in the presence of a catalytically effective amount of an amination catalyst and at a temperature and pressure sufficient to form said polyalkylene polyamines; and

(b) before and/or during reation step (a), adjusting the amount of said alkanolamine, said ammonia, said aminoalkylalkanolamine and said alkyleneamine in said feed, relative to each other, whereby said specified molecular range and distribution of said polyalkylene polyamines formed in said reaction (a) is obtained.

85. The process as claimed in Claim 84 wherein the adjustment in step (b) is achieved by changing the relative amount of said alkanolamine.

86. The process as claimed in Claim 84 wherein the adjustment in step (b) is achieved by changing the relative amount of said alkylenediamine.

87. The process as claimed in Claim 84 wherein said alkanolamine is ethanolamine, said alkyleneamine is ethylenediamine, said aminoalkylalkanolamine is aminoethylethanolamine and said reactant (iv) is ammonia.

88. The process as claimed in Claim 84 wherein the mole ratio of noncyclic polyalkylene polyamines to cyclic polyalkylene polyamines is increased by increasing the reactor pressure by increasing the amount of ammonia and/or hydrogen and/or water in the reactor feed.

89. The process as claimed in Claim 88 wherein said amination catalyst in step (b) is a reductive amination catalyst.

90. The process as claimed in Claim 88 wherein said amination catalyst in step (b) is a phosphorus-containging catalyst.

91. Process for the production of higher molecular weight polyalkylene polyamines from a feed which includes oxygen-containing higher molecular weight polyalkylene polyamines, comprising contacting said oxygen-containing higher molecular weight polyalkylene polyamines with an alkyleneamine having at least two amino groups in the presence of a catalytically effective amount of an amination catalyst at a temperature and pressure effective to produce said higher molecular polyalkylene polyamines.

92. The process as claimed in Claim 91 wherein ammonia is added to said reaction step.

93. The process as claimed in Claim 91 wherein said higher molecular weight polyalkylene polyamines have at least four amino groups.

94. The process as claimed in Claim 91 wherein said oxygen-containing higher molecular weight polyethylene polyamines are hydroxyl-containing higher molecular weight polyethylene polyamines.

95. The process as claimed in Claim 94 wherein said oxygen-containing higher molecular weight polyethylene polyamines feed is at least part of the bottoms portions obtained from a distillation column for separating at least part of diethylenetriamine from said bottoms portion.

96. The process as claimed in Claim 94 wherein at least part of the reaction mixture from said reaction step are separated using a batch still into a stream containing said at least part of higher molecular weight polyethylene polyamines and a stream at least part of containing water and ethylenediamine.

97. The process as claimed in Claim 96 wherein at least part of said stream containing water and ethylenediamine is recycled to said reaction step.

98. The process as claimed in Claim 81 wherein said amination catalyst in step (b) is a reductive amination catalyst.

99. The process as claimed in Claim 91 wherein said amination catalyst in step (b) is a phosphorus-containing catalyst.

FIG.1

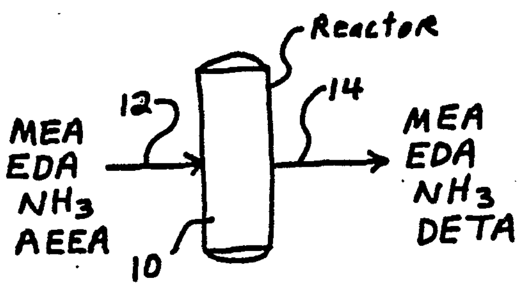
Reactor
12
14
MEA
EDA
NH3
AEEA
10
MEA
EDA
NH3
DETA

FIG.2

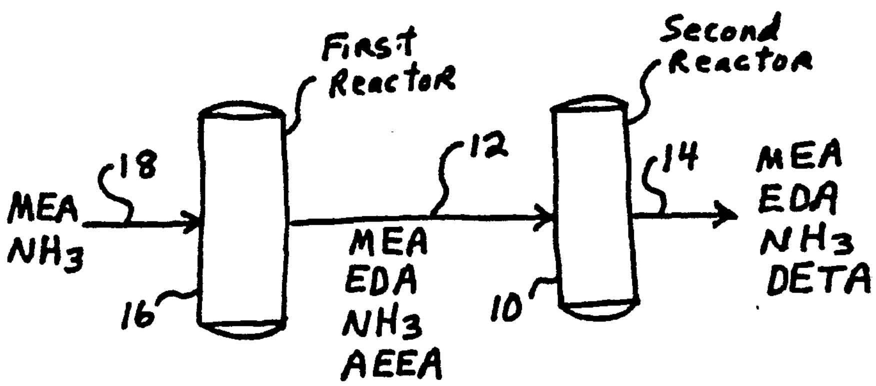
First Reactor
Second Reactor
18
MEA
NH3
16
12
MEA
EDA
NH3
AEEA
10
14
MEA
EDA
NH3
DETA

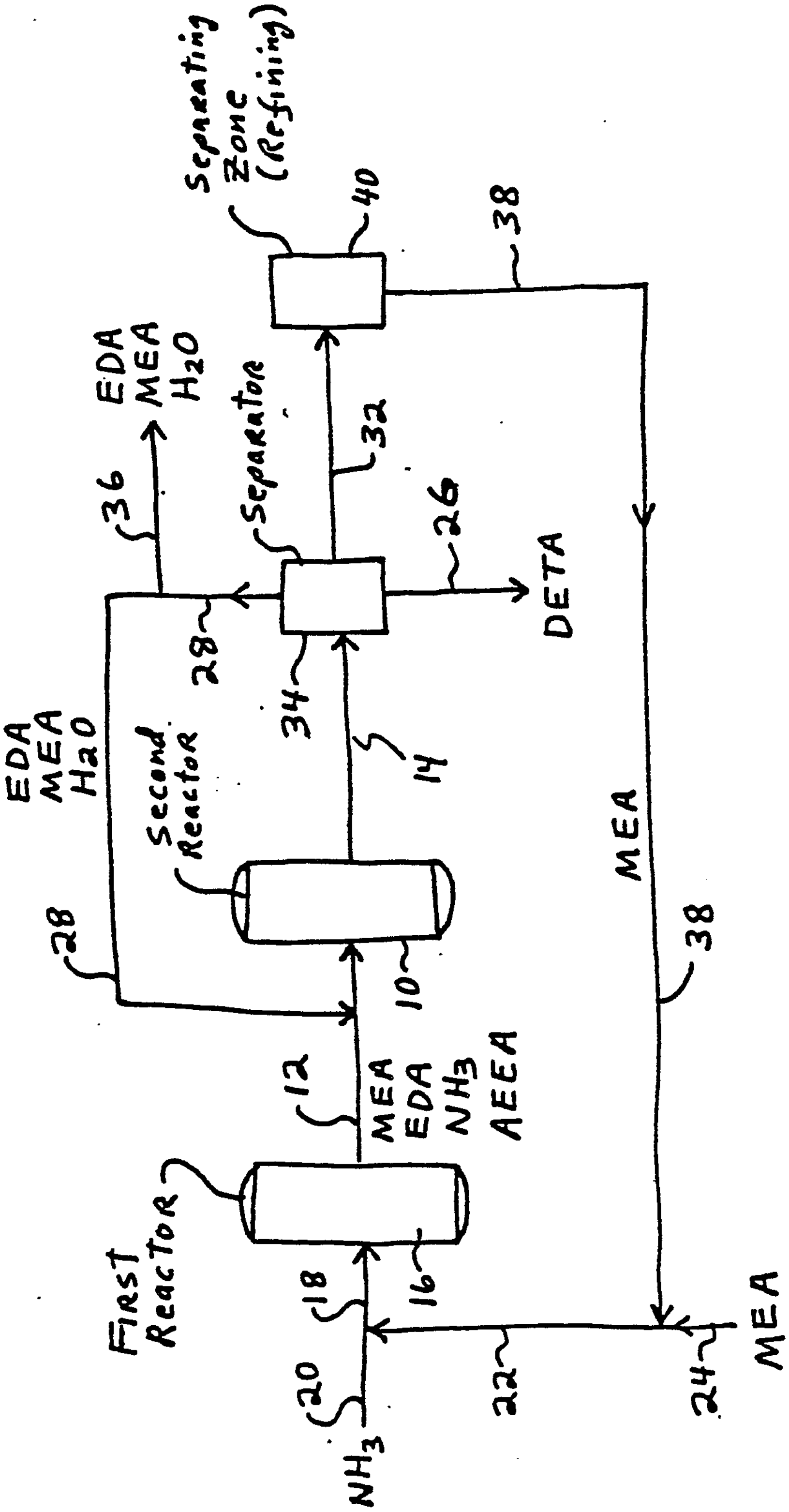
Separating Zone (Refining)
40
38
EDA
MEA
$H_2O$
Separator
32
36
26
DETA
28
34
14
EDA
MEA
$H_2O$
Second Reactor
MEA
38
28
10
12
MEA
EDA
$NH_3$
AEEA
First Reactor
18
16
20
$NH_3$
22
24
MEA

FIG. 3